# EUROPEAN PATENT APPLICATION

(11) **EP 2 743 699 A1**
(43) Date of publication of application: **18.06.2014**
(21) Application number: 11870979.9
(22) Date of filing: 12.08.2011
(51) Int. Cl.: G01N 33/68, C07K 16/14, G01N 33/15, G01N 33/50, G01N 33/53, G01N 33/531

(54) **METHOD FOR TESTING FOR, PREVENTING, AND TREATING INFECTIOUS DISEASE ASPERGILLUS FUMIGATUS, AND COMPOSITION**

(71) Applicant: National Institute of Infectious Diseases, Shinjuku-ku Tokyo 162-8640 (JP); Medical & Biological Laboratories Co., Ltd., Nagoya-shi, Aichi 460-0008 (JP)
(72) Inventor: MIYAZAKI, Yoshitsugu, Tokyo 162-8640 (JP); YAMAGOE, Satoshi, Tokyo 162-8640 (JP); KAJIKAWA, Masunori, Komagane-shi Nagano 399-4117 (JP); SUGIURA, Masahito, Komagane-shi Nagano 399-4117 (JP); ITOH, Reiko, Komagane-shi Nagano 399-4117 (JP); KUMAGAI, Hirotaka, Komagane-shi Nagano 399-4117 (JP)
(74) Representative: Cohausz & Florack
(86) International application number: PCT/JP2011/068454
(87) International publication number: WO 2013/024517

(57) **Abstract**

As a result of the analysis by an SST-REX method so as to identify a target molecule for treating and testing an *Aspergillus fumigatus* infection, a YMAF1 protein has been found out, which is mainly localized in the cell wall of *Aspergillus fumigatus.* Moreover, it has been found out that YMAF1 protein-deficient *Aspergillus fumigatus* has reduced spore-forming ability and pathogenicity. Further, it has been found out that the survival rate of experimental mice having aspergillosis (invasive *Aspergillus* model mice) is improved by preparing and administering an antibody against the YMAF1 protein. Furthermore, it has been found out that *Aspergillus fumigatus* can be detected with a favorable sensitivity by an ELISA system using the antibody.

## Description

### [Technical Field]

The present invention relates to methods for testing, preventing, and treating an *Aspergillus fumigatus* infection by targeting a YMAF1 (YPD medium associated major antigen of *Aspergillus fumigatus* 1) protein of *Aspergillus fumigatus,* and a molecule used in the methods. Moreover, the present invention relates to a screening method for a compound for testing, preventing, and treating the infectious disease by targeting the YMAF1 protein.

### [Background Art]

*Aspergillus fumigatus (A. fumigatus)* is a major causative fungus of deep mycoses such as chronic necrotizing pulmonary aspergillosis (CNPA). Patients who are immunodeficient due to organ transplantation, anti-cancer agent administration, HIV infection, or the like are susceptible to an opportunistic infection with *A. fumigatus* at medical sites. With chronic obstructive pulmonary disease (COPD) or the like, *A. fumigatus* causes severe symptoms, sometimes leading to even death.

*A. fumigatus* causes deep mycosis most among the causative fungi. Other causative fungi include *Aspergillus flavus* *(A. flavus*), *Aspergillus niger (A. niger), Aspergillus nidulans (**A. nidulans*), *Aspergillus terreus (A. terreus),* and fungi of other species such as *Candida albicans (C. albicans), Cryptococcus neoformans (Cryptococcus neoformans),* and zygomycetes. However, their infection mechanisms and virulence factors are hardly elucidated.

Meanwhile, a galactomannan antigen detection system currently used for early diagnosis of aspergilloses such as chronic necrotizing pulmonary aspergillosis and invasive aspergillosis (IA) has a sensitivity of approximately 80% for patients having hematological malignant diseases. However, the detection system has a problem that both of the sensitivity and specificity are low for other underlying diseases. Further, the detection system, for example, cannot distinguish surface carbohydrate antigens from those of other species, and hence cannot be said to always have satisfactory detection specificity and detection sensitivity. Furthermore, although the definitive diagnosis includes means such as tissue biopsy and culture test, these means also have problems for example as follows: there is a case where it is difficult to perform such a diagnosis depending on the state and so forth of a patient; a period of approximately several weeks is required for the culturing, so that it must take a lot of time to obtain the test result; furthermore, the positive rate is low in the culture test on clinical specimens. In such circumstances, even if a symptom believed to be of deep mycosis is observed for example in surgical or equivalent sites, the fungus cannot be identified immediately, hence bringing about a problem that it is difficult to determine an appropriate treatment method without extensive experiences and so on.

Moreover, in the current mycosis treatment, small molecule drugs such as mainly amphotericin B and micafungin are used depending on the fungal species, symptom, and so forth. However, since deep mycosis patients are immunodeficient, these therapeutic drugs have been administered at high doses, making the drugs less effective. This result s in a problem in some cases that the therapeutic effect cannot be obtained as expected, or similar problems.

From the foregoing, the establishment of early diagnosis and treatment methods for aspergilloses, which reflect the actual condition of the infection better and are capable of demonstrating the therapeutic effect, has been sought.

As an example, novel molecularly-targeted therapy, diagnosis method, or the like, which use an antibody against fungi, is conceivable as means for solving these problems. Until now, various extracellular antigen molecules of the genus *Aspergillus* have been identified (PTL 1), and also a treatment method has been developed, which uses an anti-fungal antibody, or a combination of the anti-fungal antibody with a small molecule drug (PTL 2). However, no antibody having a therapeutic effect specific to fungi, particularly deep mycosis, has been published so far.

Meanwhile, when a cell, a surface portion, and the like of a fungus are used as antigens so as to develop monoclonal antibodies, a lot of such antibodies produced tend to be against a carbohydrate antigen on the cell surface. Nevertheless, in consideration of the situation in the antibody drug development and the like so far, it is not to be expected that such antibodies against a carbohydrate antigen have therapeutic effect and action *in vivo.* Further, while the genome of *Aspergillus fumigatus* has been already analyzed, many genes are still defined as genes encoding conserved hypothetical proteins. Accordingly, although the existence of a target molecule with an unknown function is expected, the analysis at the protein level is hardly in progress, and no target molecule contributing to the establishment of early diagnosis and treatment methods for mycoses, particularly an *Aspergillus fumigatus* infection, has been developed yet at present (NPL 1).

### [Citation List]

### [Patent Literature]

[PTL 1] International Application Japanese-Phase Publication No. 2007-535897
[PTL 2] International Application Japanese-Phase Publication No. 2007-533716

### [Non Patent Literature]

[NPL 1] William C. et al., "Genomic sequence of the pathogenic and allergenic filamentous fungus Aspergillus fumigatus," Nature, 2005, vol. 438, no. 7071, pp. 1151 to 1156

### [Summary of Invention]

### [Technical Problems]

The present invention has been made in view of the problems of the conventional techniques. An object of the present invention is to identify a target molecule of early diagnosis and treatment for an *Aspergillus fumigatus* infection, and to provide a method for testing the infectious disease by targeting the molecule, and a composition for the testing. Another object of the present invention is to provide methods for preventing and treating the infectious disease by targeting the molecule, and a composition for the prevention and treatment. Still another object of the present invention is to provide a screening method for a compound useful in testing, preventing, and treating the infectious disease.

### [Solutions to Problems]

The present inventors thought that among extracellular proteins of *Aspergillus fumigatus,* there would be an extracellular protein involved in the pathogenicity and serving as an excellent target of diagnostic and therapeutic drugs. First, the inventors comprehensively identified extracellular proteins such as membrane proteins, cell wall proteins, and secretory proteins of *Aspergillus fumigatus,* using a signal sequence trap (SST-REX) method capable of comprehensively identifying the extracellular proteins. Further, the analysis was performed focusing on a YMAF1 (YPD medium associated major antigen of *Aspergillus fumigatus* 1) protein believed to be expressed in a relatively large amount among the identified extracellular proteins.

As a result, it was found out that: the YMAF1 protein was a protein localized in the cell wall, cell membrane, or periplasm of *Aspergillus fumigatus;* a YMAF1 gene-deficient *Aspergillus fumigatus* strain had a reduced spore-forming ability under a specific temperature condition; furthermore, the strain had a reduced pathogenicity.

From such characteristics of the YMAF1 protein, an antibody against the protein was expected to be a molecule useful for testing, preventing and treating aspergilloses. Accordingly, the present inventors next prepared and examined the antibody against the YMAF1 protein. As a result, it was revealed that: *Aspergillus fumigatus* was detected with a favorable sensitivity by ELISA utilizing the antibody; and administering the antibody improved the survival rate of experimental mice having aspergillosis (invasive *Aspergillus* model mice).

According to the above results, the present inventors have found out that the YMAF1 protein is an excellent target molecule for testing, preventing, and treating aspergilloses. This discovery has led to the completion of the present invention.

Thus, the present invention relates to methods for testing, preventing, and treating an *Aspergillus fumigatus* infection, and a molecule used in the methods, as well as a screening method for a compound for testing, preventing, and treating the infectious disease. More specifically, the present invention provides the following inventions.
(1) A method for testing an *Aspergillus fumigatus* infection, comprising a step of detecting a presence of a YMAF1 protein in a biological sample separated from a subject.
(2) The method according to (1), wherein the presence of the YMAF1 protein is detected using an antibody against the YMAF1 protein.
(3) A composition for testing an *Aspergillus fumigatus* infection, comprising an antibody against a YMAF1 protein.
(4) A method for preventing or treating an *Aspergillus fumigatus* infection, comprising a step of administering an antibody against a YMAF1 protein.
(5) A composition for preventing or treating an *Aspergillus fumigatus* infection, comprising an antibody against a YMAF1 protein.
(6) A screening method for a compound for testing, preventing, or treating an *Aspergillus fumigatus* infection, the method comprising the steps of:
   bringing a test compound into contact with any one of a YMAF1 protein and a portion thereof; and
   selecting a compound bound to any one of the YMAF1 protein and the portion thereof.
(7) An antibody capable of recognizing a region comprising the amino acid sequence of SEQ ID NO: 33 in a YMAF1 protein.
(8) An antibody according to any one of the following (a) to (c):
   (a) an antibody capable of binding to a YMAF1 protein, and comprising
      a light chain variable region including amino acid sequences of SEQ ID NOs: 1 to 3 or the amino acid sequences in at least any one of which one or more amino acids are substituted, deleted, added, and/or inserted, and
      a heavy chain variable region including amino acid sequences of SEQ ID NOs: 4 to 6 or the amino acid sequences in at least any one of which one or more amino acids are substituted, deleted, added, and/or inserted;
   (b) an antibody capable of binding to the YMAF1 protein, and comprising
      a light chain variable region including amino acid sequences of SEQ ID NOs: 7 to 9 or the amino acid sequences in at least any one of which one or more amino acids are substituted, deleted, added, and/or inserted, and
      a heavy chain variable region including amino acid sequences of SEQ ID NOs: 10 to 12 or the amino acid sequences in at least any one of which one or more amino acids are substituted, deleted, added, and/or inserted; and
   (c) an antibody capable of binding to the YMAF1 protein, and comprising
      a light chain variable region including amino acid sequences of SEQ ID NOs: 13 to 15 or the amino acid sequences in at least any one of which one or more amino acids are substituted, deleted, added, and/or inserted, and
      a heavy chain variable region including amino acid sequences of SEQ ID NOs: 16 to 18 or the amino acid sequences in at least any one of which one or more amino acids are substituted, deleted, added, and/or inserted.
(9) An antibody according to any one of the following (a) to (c):
   (a) an antibody capable of binding to a YMAF1 protein, and comprising
      a light chain variable region including the amino acid sequence of SEQ ID NO: 20, the amino acid sequence from which a signal sequence is removed, or at least any one of these amino acid sequences in which one or more amino acids are substituted, deleted, added, and/or inserted, and
      a heavy chain variable region including the amino acid sequence of SEQ ID NO: 22, the amino acid sequence from which a signal sequence is removed, or at least any one of these amino acid sequences in which one or more amino acids are substituted, deleted, added, and/or inserted;
   (b) an antibody capable of binding to the YMAF1 protein, and comprising
      a light chain variable region including the amino acid sequence of SEQ ID NO: 24, the amino acid sequence from which a signal sequence is removed, or at least any one of these amino acid sequences in which one or more amino acids are substituted, deleted, added, and/or inserted, and
      a heavy chain variable region including the amino acid sequence of SEQ ID NO: 26, the amino acid sequence from which a signal sequence is removed, or at least any one of these amino acid sequences in which one or more amino acids are substituted, deleted, added, and/or inserted; and
   (c) an antibody capable of binding to the YMAF1 protein, and comprising
      a light chain variable region including the amino acid sequence of SEQ ID NO: 28, the amino acid sequence from which a signal sequence is removed, or at least any one of these amino acid sequences in which one or more amino acids are substituted, deleted, added, and/or inserted, and
      a heavy chain variable region including the amino acid sequence of SEQ ID NO: 30, the amino acid sequence from which a signal sequence is removed, or at least any one of these amino acid sequences in which one or more amino acids are substituted, deleted, added, and/or inserted.

It should be noted that the sequence per se of the YMAF1 gene according to the present invention is disclosed in the specification of US Patent No. 7504490 as a result of comprehensively analyzing the gene expressed in *Aspergillus fumigatus.* Nonetheless, the existence and function of a protein encoded by the YMAF1 gene have not been revealed.

### [Advantageous Effects of Invention]

The present invention makes it possible to provide a method for testing an *Aspergillus fumigatus* infection, the method being capable of detecting *Aspergillus fumigatus* with high specificity and sensitivity, and a composition for the testing. Moreover, it becomes possible to provide methods for preventing and treating an *Aspergillus fumigatus* infection, and a composition for the prevention and treatment. Furthermore, it becomes possible to provide a screening method for a compound useful in these methods, and an antibody useful in these methods.

### [Brief Description of Drawings]

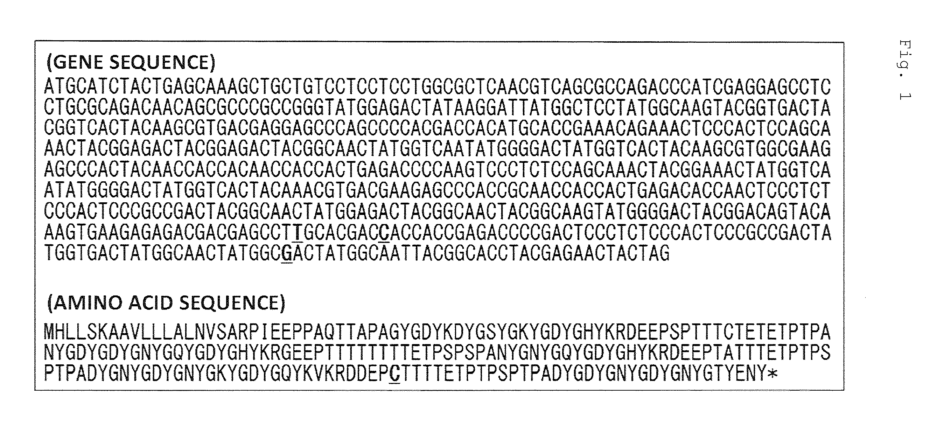

[Fig. 1] Fig. 1 is a figure showing a base sequence (gene sequence) of a YMAF1 (YPD medium associated major antigen of *Aspergillus fumigatus* 1) gene of *Aspergillus fumigatus,* and an amino acid sequence of a protein encoded by the gene. Note that, in the figure, bases and an amino acid, which are underlined, indicate that the base sequence and the amino acid sequence are different from a base sequence specified by GenBank Accession No: XM_726394.1 and the amino acid sequence specified by GenBank Accession No: XP_731487.1.

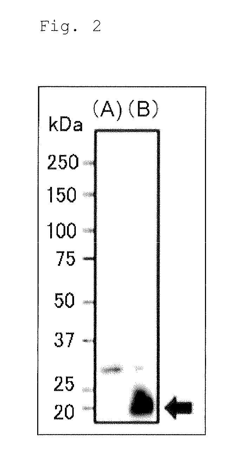

[Fig. 2] Fig. 2 is a photograph for illustrating the result of expressing in a yeast the YMAF1 protein having a HA tag added thereto, purifying a culture supernatant of the yeast by immunoprecipitation using an anti-HA antibody, and analyzing the resultant by western blotting using an anti-HA antibody. Note that, in the figure, (A) shows the result of expressing in the yeast a vector encoding only the HA tag (pADH-HA expression vector) (negative control), while (B) shows the result of expressing in the yeast a vector encoding the YMAF1 protein having the HA tag added thereto.

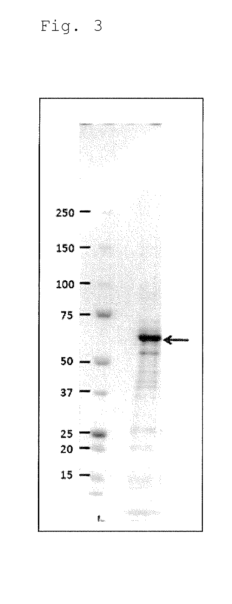

[Fig. 3] Fig. 3 is a photograph for illustrating the result of expressing a fusion protein between GST and YMAF1 in *Escherichia coli,* and analyzing a soluble fraction of the *Escherichia coli* by SDS-PAGE and CBB staining. Note that, in the figure, the band indicated by the arrow is derived from the fusion protein between GST and YMAF1.

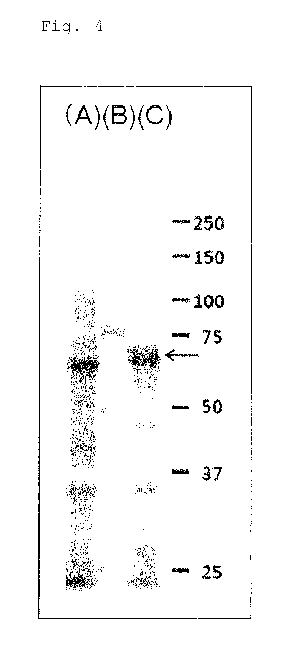

[Fig. 4] Fig. 4 is a photograph for illustrating the result of expressing the fusion protein between GST and YMAF1 in *Escherichia coli,* and analyzing the soluble fraction and an insoluble fraction of the *Escherichia coli* by SDS-PAGE and CBB staining. Note that, in the figure, (A) shows the result of analyzing the soluble fraction of the *Escherichia coli,* (B) shows the result of a marker migration, and (C) shows the result of analyzing a soluble fraction obtained by further treating the insoluble fraction of the *Escherichia coli* with 8 M urea. In addition, the band indicated by the arrow is derived from the fusion protein between GST and YMAF1.

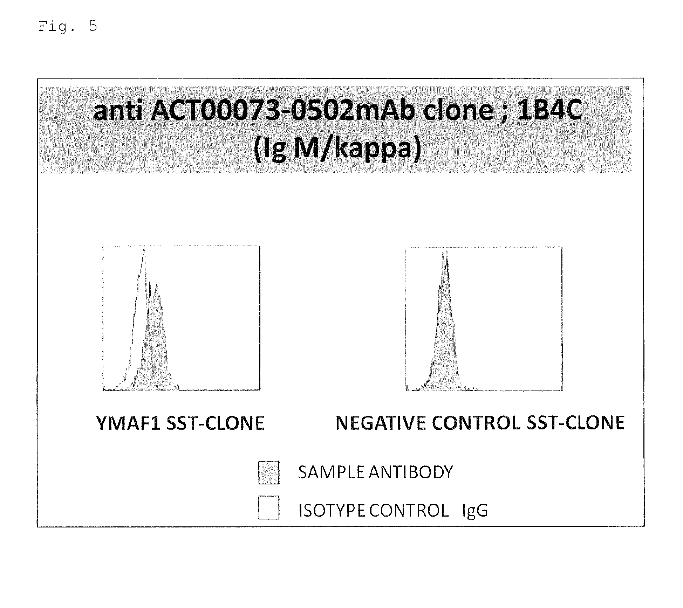

[Fig. 5] Fig. 5 shows graphs for illustrating the reactivity between a 1B4C antibody and Ba/F3 cells expressing the YMAF1 gene. The reaction of the 1B4C antibody to transfectant Ba/F3 cells expressing the full-length YMAF1 gene (YMAF1 SST-clone), which are the immunogen cells, and to control Ba/F3 cells not expressing the YMAF1 gene (negative control SST-clone) was analyzed with a flow cytometer. A filled histogram part in the flow cytometer data illustrates the reaction of the sample antibody (1B4C antibody), whereas a white histogram part illustrates the reaction of negative control IgM/kappa (isotype control IgG).

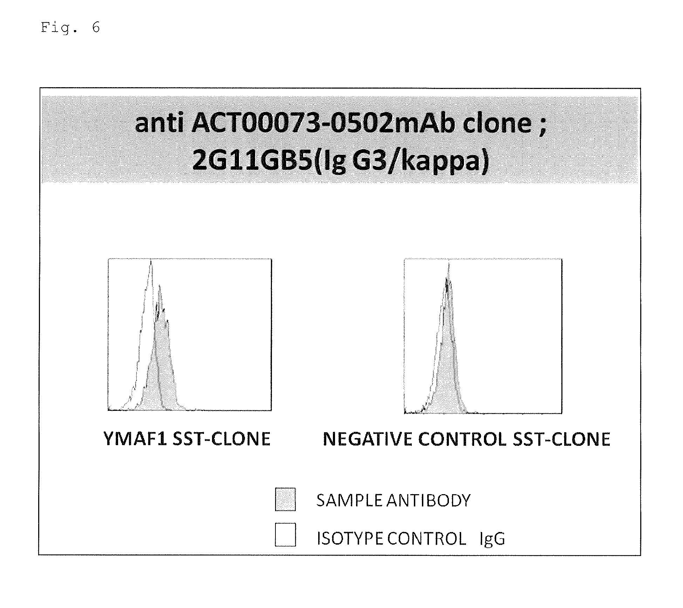

[Fig. 6] Fig. 6 shows graphs for illustrating the reactivity between a 2G11GB5 antibody and the Ba/F3 cells expressing the YMAF1 gene. The reaction of the 2G11GB5 antibody to the transfectant Ba/F3 cells expressing the full-length YMAF1 gene (YMAF1 SST-clone), which are the immunogen cells, and to the control Ba/F3 cells not expressing the YMAF1 gene (negative control SST-clone) was analyzed with the flow cytometer. A filled histogram part in the flow cytometer data illustrates the reaction of the sample antibody (2G11GB5 antibody), whereas a white histogrampart illustrates the reaction of negative control IgG3/kappa (isotype control IgG).

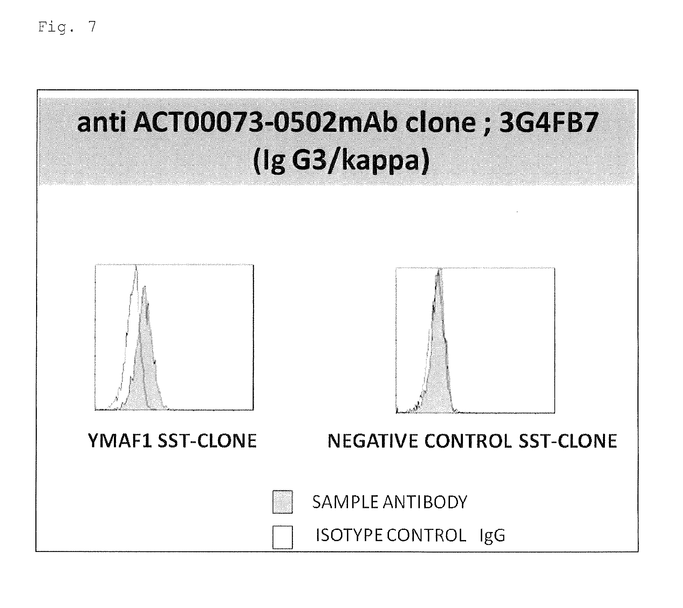

[Fig. 7] Fig. 7 shows graphs for illustrating the reactivity between a 3G4FB7 antibody and the Ba/F3 cells expressing the YMAF1 gene. The reaction of the 3G4FB7 antibody to the transfectant Ba/F3 cells expressing the full-length YMAF1 gene (YMAF1 SST-clone), which are the immunogen cells, and to the control Ba/F3 cells not expressing the YMAF1 gene (negative control SST-clone) was analyzed with the flow cytometer. A filled histogram part in the flow cytometer data illustrates the reaction of the sample antibody (3G4FB7 antibody), whereas a white histogram part illustrates the reaction of the negative control IgG3/kappa (isotype control IgG).

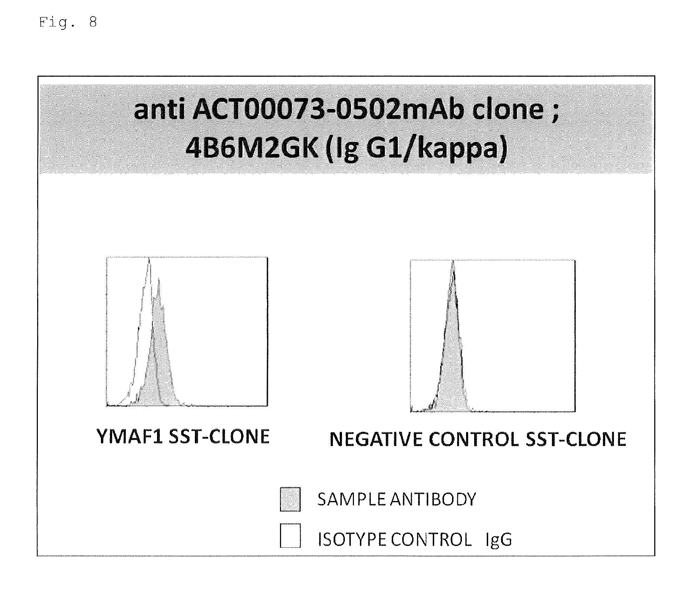

[Fig. 8] Fig. 8 shows graphs for illustrating the reactivity between a 4B6M2GK antibody and the Ba/F3 cells expressing the YMAF1 gene. The reaction of the 4B6M2GK antibody to the transfectant Ba/F3 cells expressing the full-length YMAF1 gene (YMAF1 SST-clone), which are the immunogen cells, and to the control Ba/F3 cells not expressing the YMAF1 gene (negative control SST-clone) was analyzed with a flow cytometer. A filled histogram part in the flow cytometer data illustrates the reaction of the sample antibody (4B6M2GK antibody), whereas a white histogram part illustrates the reaction of negative control IgG1/kappa (isotype control IgG).

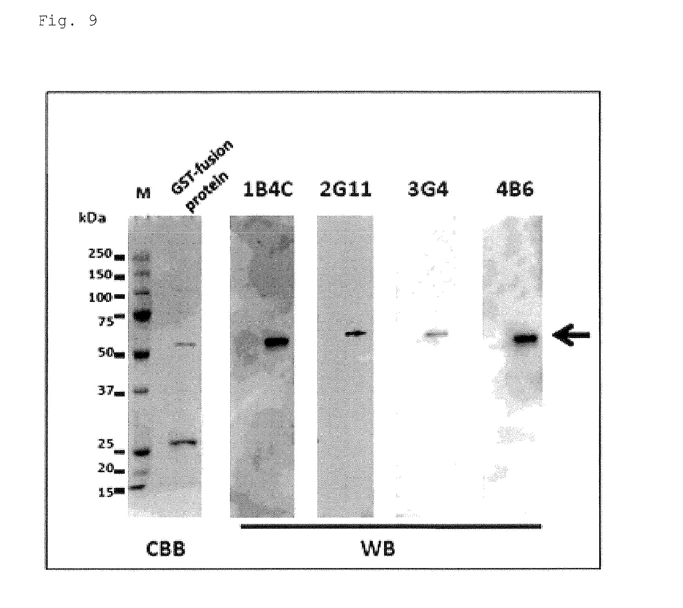

[Fig. 9] Fig. 9 shows photographs for illustrating the result of western blotting performed on the fusion protein between GST and YMAF1 produced in *Escherichia coli,* using the 1B4C antibody, the 2G11GB5 antibody, the 3G4FB7 antibody, or the 4B6M2GK antibody. Note that, in the figure, "M" shows the result of analyzing by CBB staining a marker separated by SDS-PAGE, while "GST-fusion protein" shows the result of analyzing by CBB staining the fusion protein between GST and YMAF1 separated by SDS-PAGE. Moreover, "1B4C", "2G11", "3G4", and "4B6" respectively show the results of the western blotting (WB) using the 1B4C antibody, the 2G11GB5 antibody, the 3G4FB7 antibody, and the 4B6M2GK antibody. In addition, the band indicated by the arrow is derived from the fusion protein between GST and YMAF1.

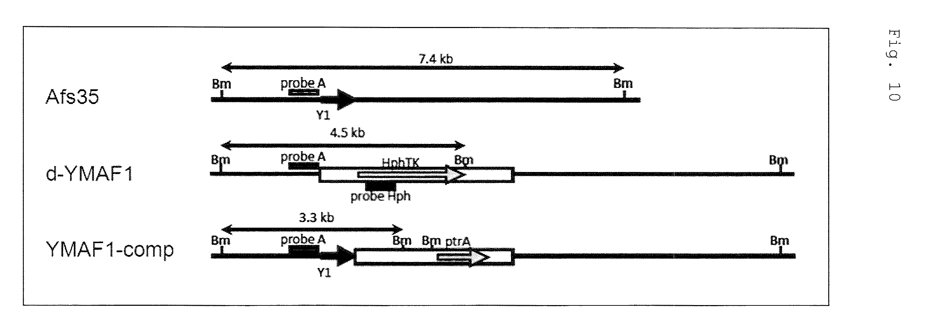

[Fig. 10] Fig. 10 is a schematic drawing showing structures of genomes in the vicinity of Y1 genes of a YMAF1 gene-disrupted strain (d-YMAF1) and a YMAF1 gene complementation strain (YMAF1-comp). Note that, in the figure, "Y1" indicates the YMAF1 gene, "probe A" indicates a probe specific to an upstream region of the YMAF1 gene (region at positions 136 to 600 of a base sequence of SEQ ID NO: 53 (genomic sequence encoding the YMAF1 protein)), "HphTK" indicates a gene encoding a fusion protein between hygromycin phosphotransferase (a protein comprising the amino acid sequence of SEQ ID NO: 56) and human herpes thymidine kinase, and "probe Hph" indicates a probe specific to a hygromycin phosphotransferase gene. Note that the sequence of a DNA encoding the hygromycin phosphotransferase is shown in SEQ ID NO: 55. Moreover, the region, to which the "probe Hph" hybridizes, is a region at positions 218 to 755 of the base sequence of SEQ ID NO: 55. Further, "ptrA" indicates a pyrithiamine resistance gene, and "Bm" indicates a recognition site of a restriction enzyme BamH1. Furthermore, "Afs35" indicates the structure of a genome of a parental strain that served as the basis of the YMAF1 gene-disrupted strain and the YMAF1 gene complementation strain.

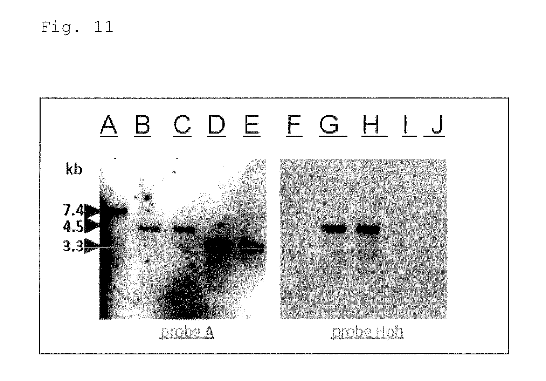

[Fig. 11] Fig. 11 shows photographs for illustrating the result of analyzing the genomes of the YMAF1 gene-disrupted strain and the YMAF1 gene complementation strain by Southern hybridization. Note that, in the figure, "probe A" shows the analysis result by the Southern hybridization using the probe specific to the upstream region of the YMAF1 gene, and "probe Hph" shows the analysis result by the Southern hybridization using the probe specific to the hygromycin phosphotransferase gene. Moreover,"A"and"F" show the result of analyzing the genome of the parental strain (Afs35), "B" and "G" show the result of analyzing of the genome of a YMAF1 gene-disrupted strain (d-YMAF1-5), "C" and "H" shows the result of analyzing of the genome of a YMAF1 gene-disrupted strain (d-YMAF1-7), "D" and "I" shows the result of analyzing the genome of a YMAF1 gene complementation strain (YMAF1-comp-3), and "C" and "H" shows the result of analyzing the genome of a YMAF1 gene complementation strain (YMAF1-comp-4).

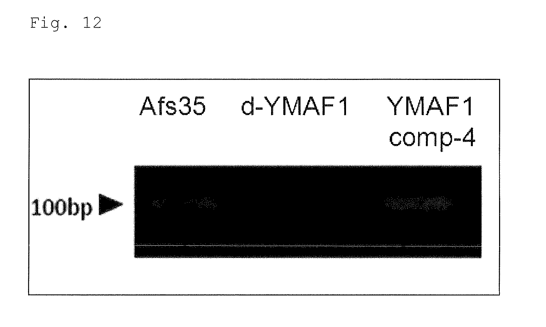

[Fig. 12] Fig. 12 is a photograph for illustrating the result of analyzing by PCR expressions of YMAF1 mRNAs in the YMAF1 gene-disrupted strain (d-YMAF1), the YMAF1 gene complementation strain (YMAF1 comp-4), and the parental strain thereof (Afs35).

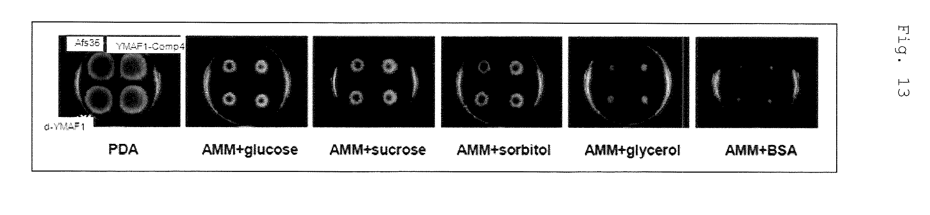

[Fig. 13] Fig. 13 shows photographs for illustrating the result of culturing at 30°C for 3 days spore solutions (1 × 10⁷ conidia/2 µl) of the YMAF1 gene-disrupted strain (d-YMAF1 (d-YMAF1-7)), the YMAF1 gene complementation strain (YMAF1-comp4), and the parental strain thereof (Afs35), which had been added to various media. Note that, in the figure, positions of colonies derived from the strains shown on a plate in "PDA" respectively correspond to positions on plates of the other media.

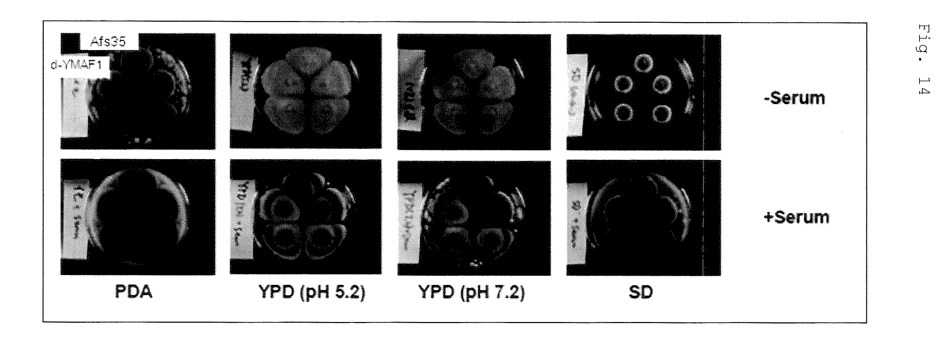

[Fig. 14] Fig. 14 shows photographs for illustrating the result of culturing at 30°C for 3 days the spore solution (1 × 10⁴ conidia/2 µl) of the YMAF1 gene-disrupted strain (d-YMAF1 (d-YMAF1-7)) and the parental strain (Afs35), which had been added to various media. Note that, in the figure, positions of colonies derived from the strains shown on a plate in "PDA" respectively correspond to positions on plates of the other media. Moreover, the upper row (-Serum) shows the result in various media not supplemented with fetal bovine serum, while the lower row (+Serum) shows the result in various media supplemented with fetal bovine serum.

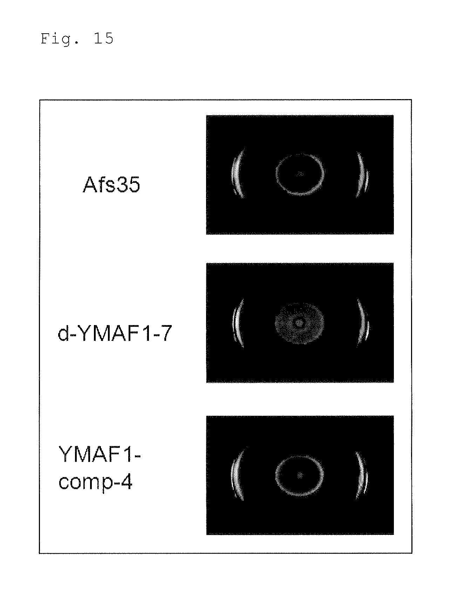

[Fig. 15] Fig. 15 shows photographs for illustrating the result of observing the colony state of the YMAF1 gene-disrupted strain (d-YMAF1-7), the YMAF1 gene complementation strain (YMAF1-comp-4), and the parental strain (Afs35), which had been cultured a 10% bovine serum-containing Spider medium.

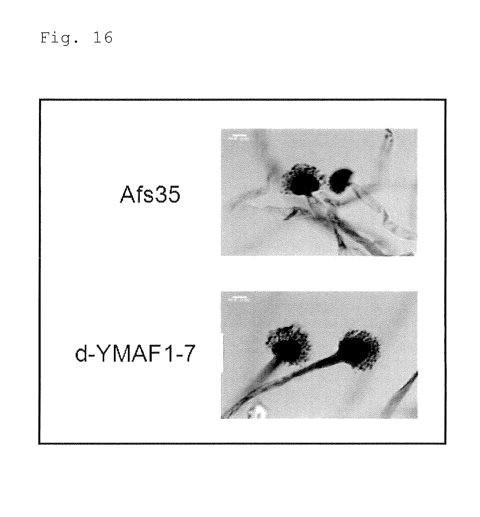

[Fig. 16] Fig. 16 shows photographs for illustrating the result of observing the form of conidial heads of the YMAF1 gene-disrupted strain (d-YMAF1-7) and the parental strain (Afs35), which had been cultured in the 10% bovine serum-containing Spider medium.

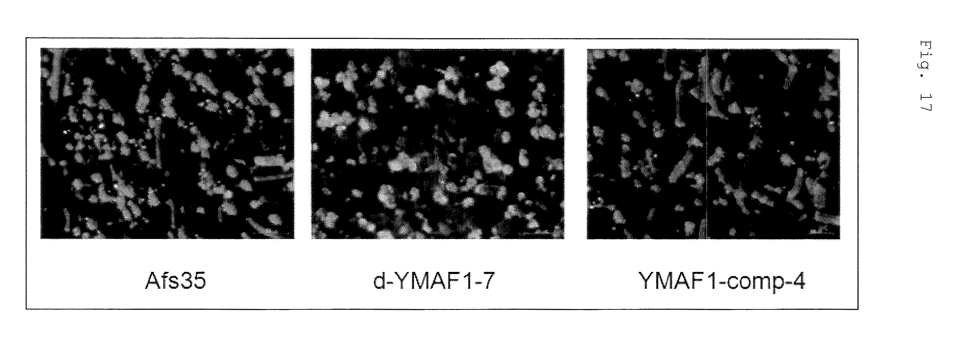

[Fig. 17] Fig. 17 shows photographs for illustrating the result of observing the state of the spore formation of the YMAF1 gene-disrupted strain (d-YMAF1-7), the YMAF1 gene complementation strain (YMAF1-comp-4), and the parental strain (Afs35), which had been grown at 25°C in a 10% bovine serum-containing a PDA medium.

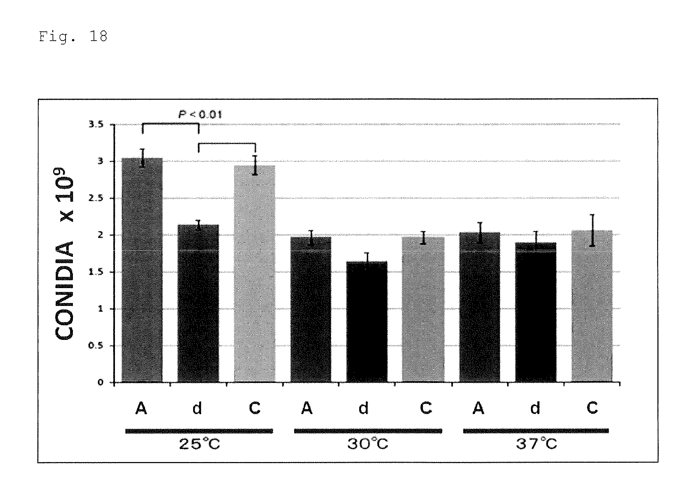

[Fig. 18] Fig. 18 is a graph showing the number of spores (vertical axis: × 10⁹ conidia) of the YMAF1 gene-disrupted strain (d: d-YMAF1-7), the YMAF1 gene complementation strain (C: YMAF1-comp-4), and the parental strain thereof (A: Afs35), which had been cultured in minimal media AMM at 25°C, 30°C, or 37°C.

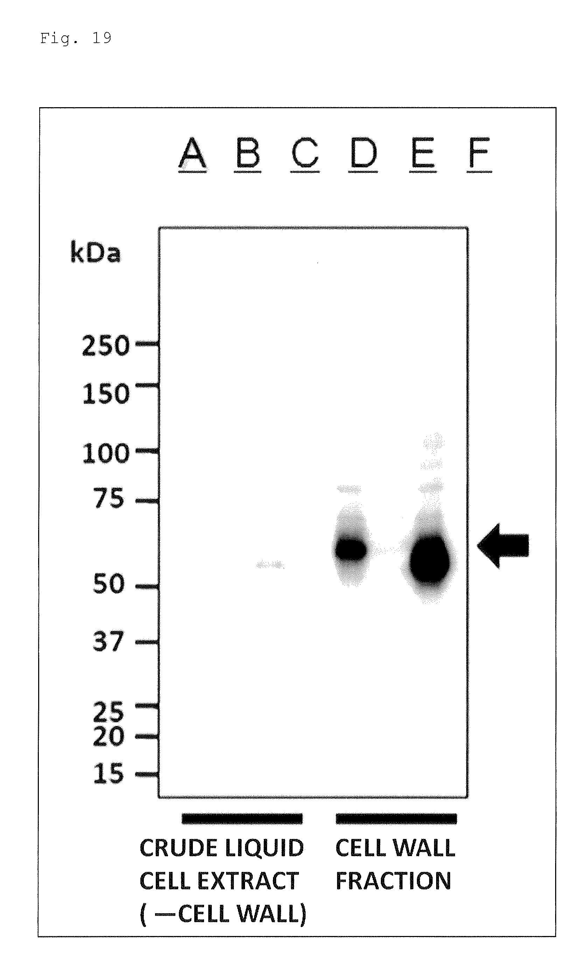

[Fig. 19] Fig. 19 is a photograph for illustrating the result of analyzing crude liquid cell extracts and cell wall fractions of the YMAF1 gene-disrupted strain, the YMAF1 gene complementation strain, and the parental strain by western blotting using an anti-YMAF1 rabbit polyclonal antibody. Note that, in the figure, "A" and "D" show the result of analyzing of a protein derived from the parental strain (Afs35), "B" and "E" show the result of analyzing of a protein derived from the YMAF1 gene-disrupted strain (d-YMAF1-7), and "C" and "F" show the result of analyzing a protein derived from the YMAF1 gene complementation strain (YMAF1-comp-4).

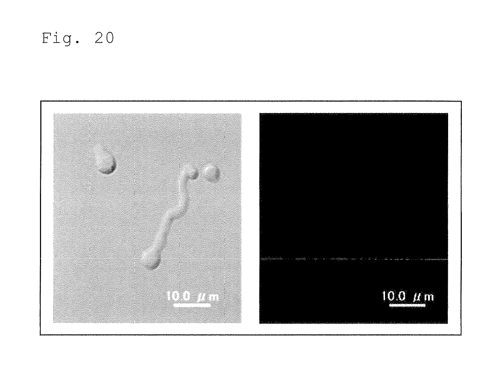

[Fig. 20] Fig. 20 shows micrographs for illustrating the result of analyzing the Afs35 strain by immunostaining using the anti-YMAF1 antibody. Note that, in the figure, the left side shows the result of bright field observation, while the right side shows the result of fluorescence observation.

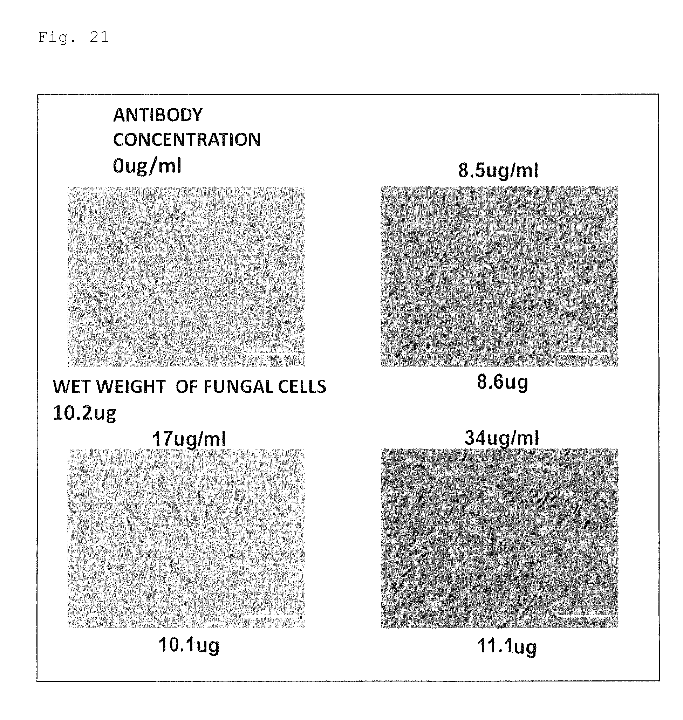

[Fig. 21] Fig. 21 shows micrographs for illustrating the result of observing the Afs35 strain was used as *Aspergillus fumigatus,* which had been cultured at 30°C for 14 hours in media having the anti-YMAF1 polyclonal antibody added at various concentrations.

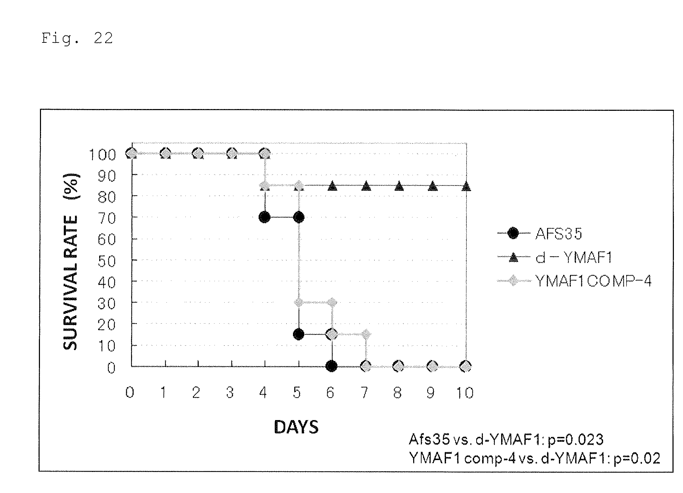

[Fig. 22] Fig. 22 is a graph showing the survival rate of experimental mice having aspergillosis (invasive *Aspergillus* model mice) to which spores of Afs35, the YMAF1 gene-deficient strain (d-YMAF1), or the YMAF1 gene complementation strain (YMAF1COMP-4) had been administered.

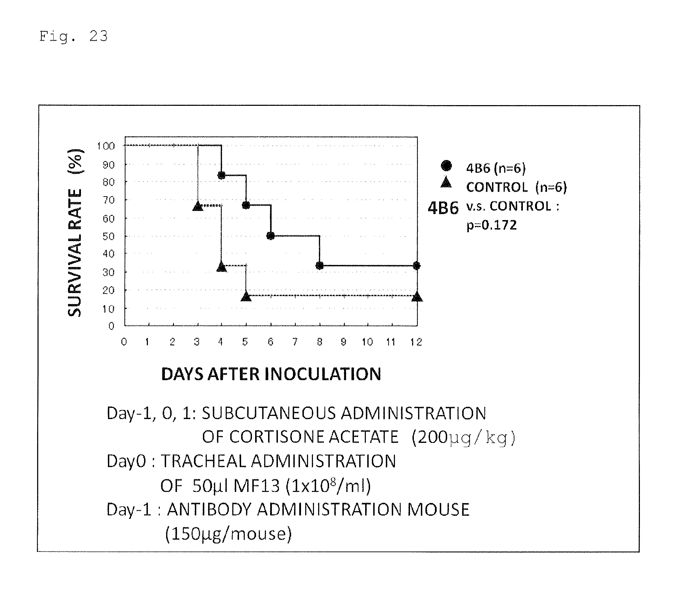

[Fig. 23] Fig. 23 is a graph showing the survival rate of experimental mice having aspergillosis (invasive *Aspergillus* model mice) to which the anti-YMAF1 protein monoclonal antibody (4B6: 4B6M2GK antibody) had been administered.

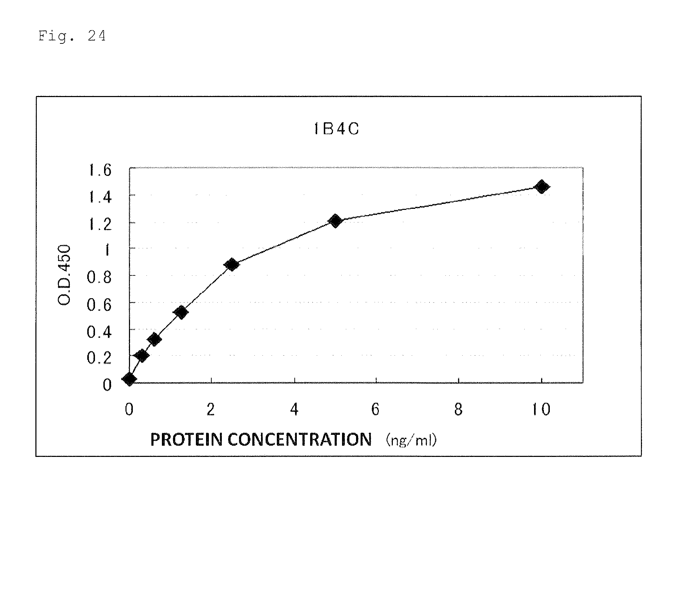

[Fig. 24] Fig. 24 is a graph showing the result of evaluating a sandwich ELISA system using the anti-YMAF1 monoclonal antibody (1B4C: 1B4C monoclonal antibody) as a capture antibody, and using a biotinylated anti-YMAF1 polyclonal antibody as a detection antibody.

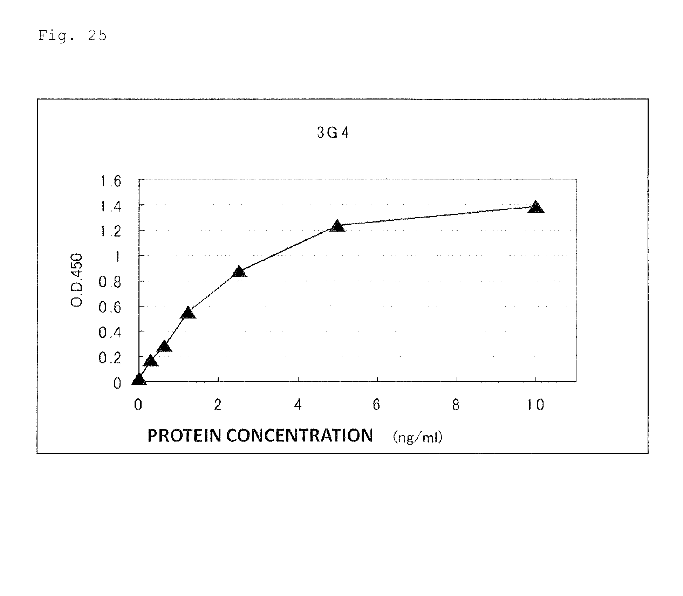

[Fig. 25] Fig. 25 is a graph showing the result of evaluating a sandwich ELISA system using the anti-YMAF1 monoclonal antibody (3G4: 3G4FB7 monoclonal antibody) as a capture antibody, and using the biotinylated anti-YMAF1 polyclonal antibody as a detection antibody.

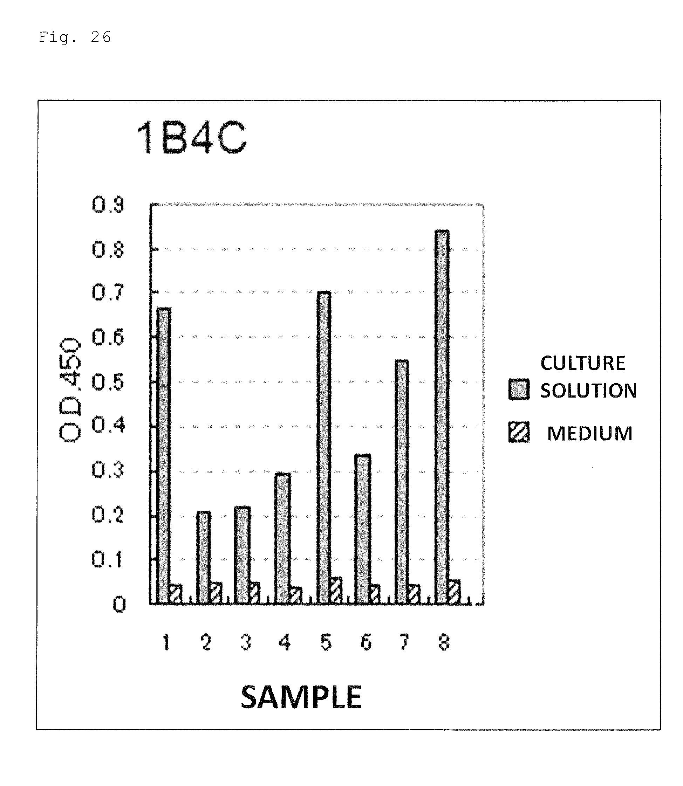

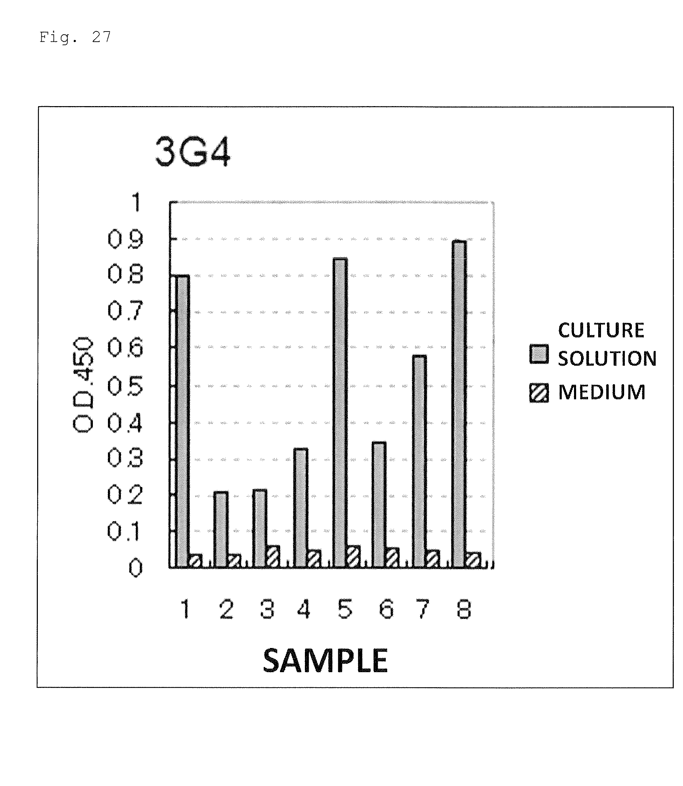

[Fig. 26] Fig. 26 is a graph showing the result of analyzing amounts of the YMAF1 proteins in culture solutions, using the YMAF1 sandwich ELISA system (the system using the 1B4C monoclonal antibody (1B4C) as the capture antibody), the culture solutions obtained by culturing Aspergillus *fumigatus* in various types of media at 30°C. Note that, in the figure, 1 shows the result of culturing in a YGmedium, 2 shows the result of culturing in a YPD pH 5.6 medium, 3 shows the result of culturing in a YPD pH 7.2 medium, 4 shows the result of culturing in a Spider medium, 5 shows the result of culturing in an AMM medium, 6 shows the result of culturing in an LB medium, 7 shows the result of culturing in a Sabouraud medium, and 8 shows the result of culturing in a SD-a.a. medium. Moreover, "medium" shows the result of analyzing only each medium (negative control) (the same applies to Fig. 27 also).
[Fig. 27] Fig. 27 is a graph showing the result of analyzing amounts of YMAF1 proteins in culture solutions, using the YMAF1 sandwich ELISA system (the system using the 3G4FB7 monoclonal antibody (3G4) as the capture antibody), the culture solutions obtained by culturing *Aspergillus fumigatus* in various types of media at 30°C.

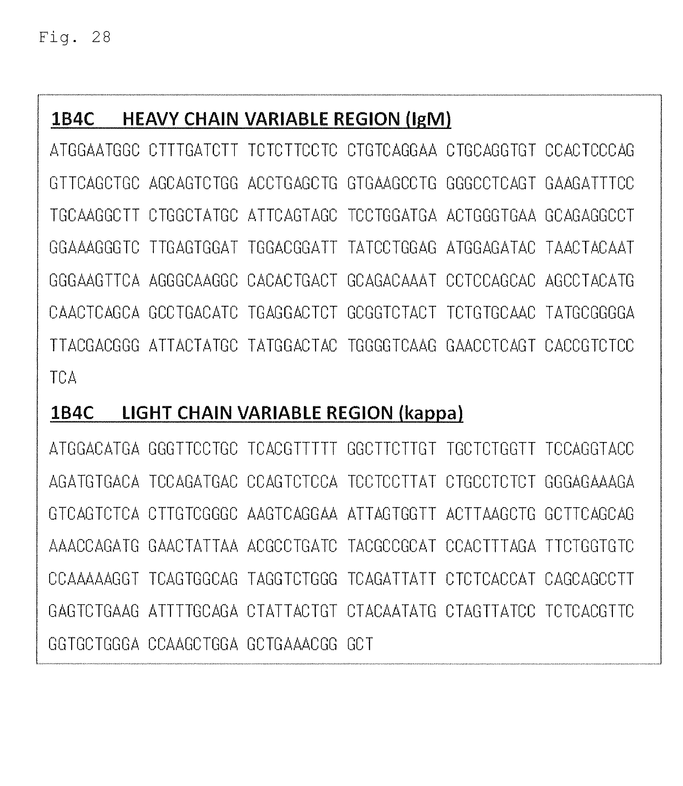

[Fig. 28] Fig. 28 is a figure showing base sequences of a heavy chain variable region and a light chain variable region of the anti-YMAF1 monoclonal antibody (1B4C monoclonal antibody).

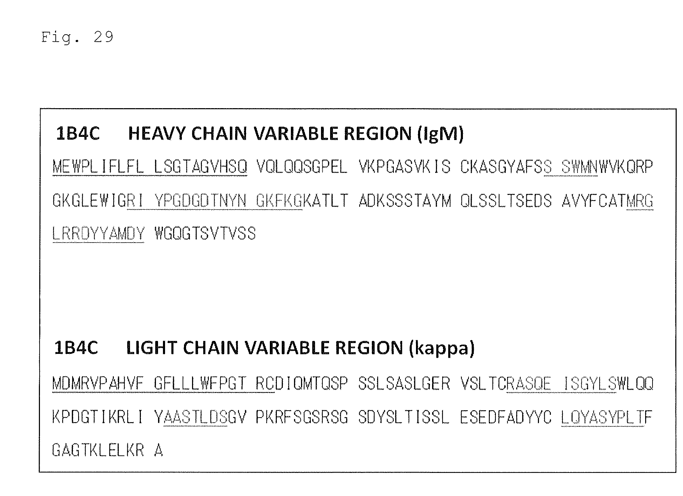

[Fig. 29] Fig. 29 is a figure showing amino acid sequences of the heavy chain variable region and the light chain variable region of the anti-YMAF1 monoclonal antibody (1B4C monoclonal antibody). Note that the underlined amino acid sequences respectively indicate a signal sequence, CDR1, CDR2, and CDR3 from the N-terminal side.

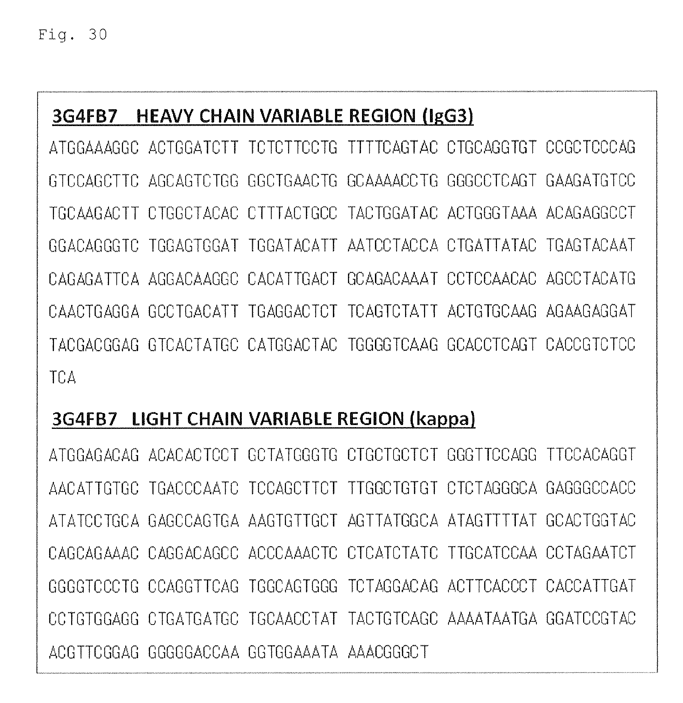

[Fig. 30] Fig. 30 is a figure showing base sequences of a heavy chain variable region and a light chain variable region of the anti-YMAF1 monoclonal antibody (3G4FB7 monoclonal antibody).

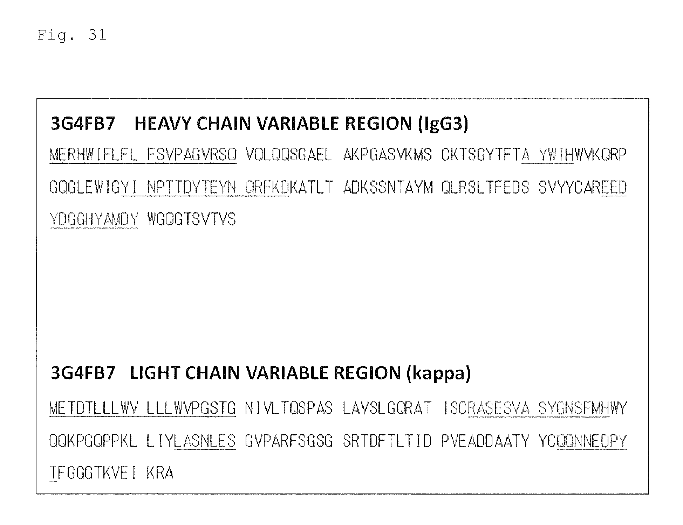

[Fig. 31] Fig. 31 is a figure showing amino acid sequences of the heavy chain variable region and the light chain variable region of the anti-YMAF1 monoclonal antibody (3G4FB7 monoclonal antibody). Note that the underlined amino acid sequences respectively indicate a signal sequence, CDR1, CDR2, and CDR3 from the N-terminal side.

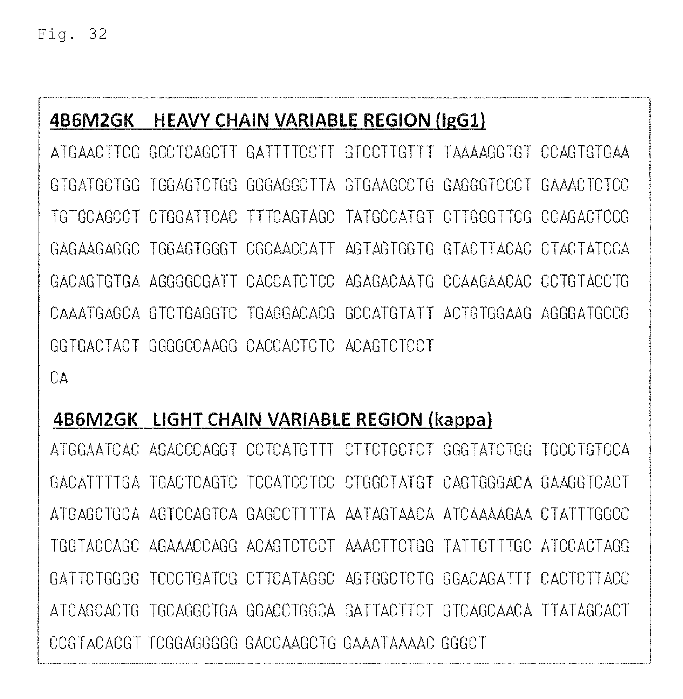

[Fig. 32] Fig. 32 is a figure showing base sequences of a heavy chain variable region and a light chain variable region of the anti-YMAF1 monoclonal antibody (4B6M2GK monoclonal antibody).

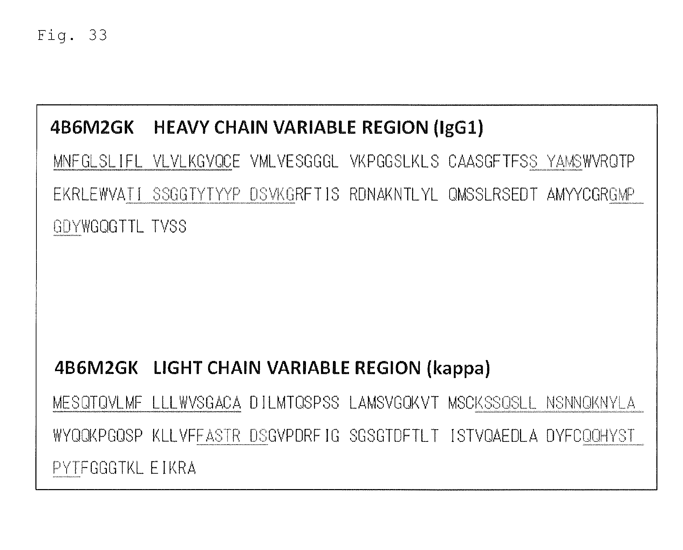

[Fig. 33] Fig. 33 is a figure showing amino acid sequences of the heavy chain variable region and the light chain variable region of the anti-YMAF1 monoclonal antibody (4B6M2GK monoclonal antibody). Note that the underlined amino acid sequences respectively indicate a signal sequence, CDR1, CDR2, and CDR3 from the N-terminal side.

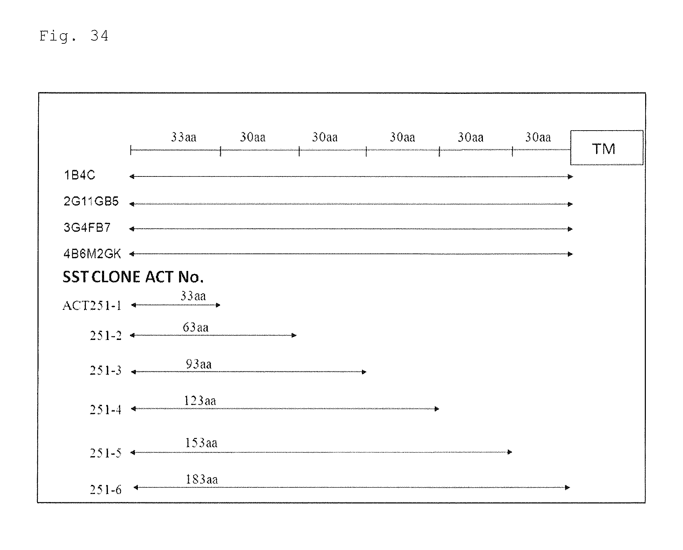

[Fig. 34] Fig. 34 is a schematic drawing the reactivities between each anti-YMAF1 monoclonal antibody (1B4C, 2G11GB5, 3G4FB7, or 4B6M2GK) and transfectant Ba/F3 cells expressing YMAF1 proteins of various lengths (SST clone: ACT251-1 to 6). Note that, in the figure, "TM" indicates a transmembrane domain of MPL.

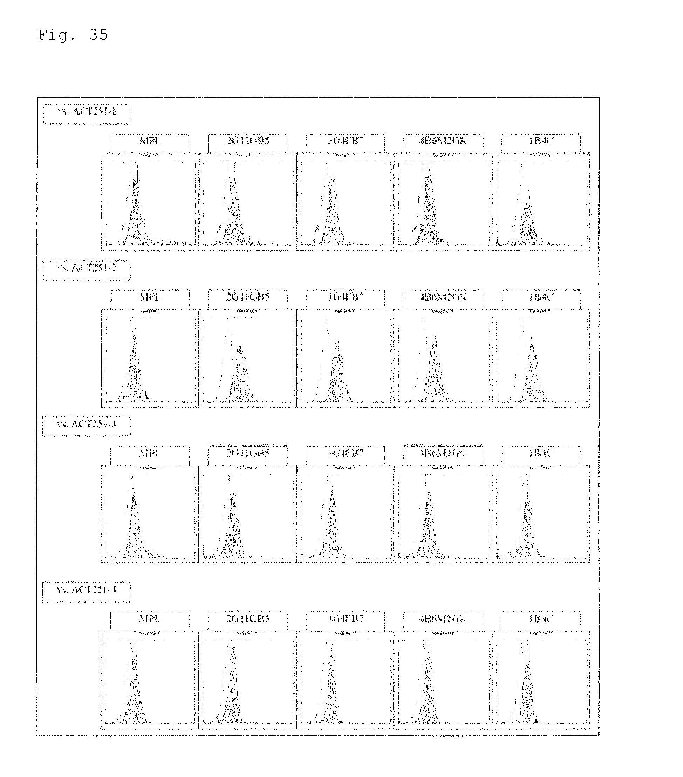

[Fig. 35] Fig. 35 shows graphs for illustrating the result of analyzing the reactivities with a flow cytometer between each anti-YMAF1 monoclonal antibody (2G11GB5, 3G4FB7, 4B6M2GK, or 1B4C) and transfectant Ba/F3 cells expressing YMAF1 proteins of various lengths (SST clone: ACT251-1 to 4).

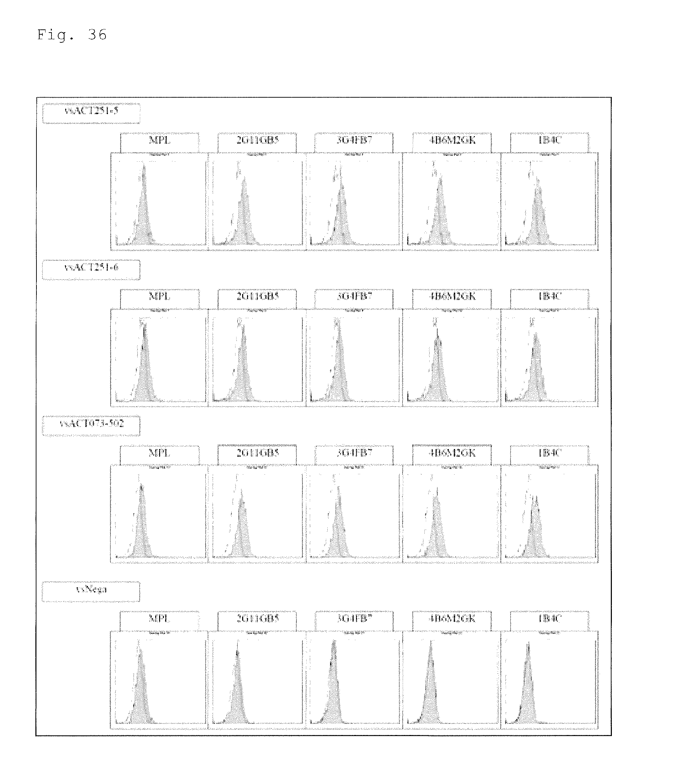

[Fig. 36] Fig. 36 shows graphs for illustrating the result of analyzing the reactivities with the flow cytometer between each anti-YMAF1 monoclonal antibody (2G11GB5, 3G4FB7, 4B6M2GK, or 1B4C) and ACT073-502 cells or transfectant Ba/F3 cells expressing YMAF1 proteins of various lengths (SST clone: ACT251-5 to 6).

### [Description of Embodiments]

### <Method for Testing Aspergillus fumigatus Infection>

As will be illustrated in Examples later, the present inventors have revealed that a YMAF1 protein is involved in the pathogenicity and spore-forming ability of *Aspergillus fumigatus.* Accordingly, on the basis of the presence of the YMAF1 protein, not only can the presence of *Aspergillus fumigatus* be detected, but also an *Aspergillus fumigatus* infection due to the pathogenicity of *Aspergillus fumigatus* can be tested. Furthermore, it has also been revealed that the protein is a protein localized in the cell wall of *Aspergillus fumigatus.* The protein dissociated from the cell wall is highly likely to be released into a serum and the like of a patient having an Aspergillus fumigatus infection. Accordingly, the testing can be conducted conveniently and efficiently with high specificity and sensitivity on the basis of the presence of the YMAF1 protein.

Thus, the present invention provides a method for testing an *Aspergillus fumigatus* infection, comprising a step of detecting a presence of a YMAF1 protein in a biological sample separated from a subject.

In the present invention, the term *"Aspergillus fumigatus* infection" refers to a disease caused by infection by *Aspergillus fumigatus (A. fumigatus).* Examples thereof include chronic pulmonary aspergillosis (CPA), invasive aspergillosis (IA), invasive pulmonary aspergillosis (IPA), and allergic bronchopulmonary aspergillosis (ABPA).

In the present invention, the term "biological sample" means a sample such as cells, tissues, organs, body fluids (for example, serum, urine), these liquids after washing (for example, bronchoalveolar lavage fluid), and the like, in which the presence of the YMAF1 protein is to be detected by the testing method of the present invention. Among these, the "biological sample" according to the present invention is preferably a serum from the viewpoint that the remainder from normal testing (routine testing) can be used as a target for the detection of the presence of the YMAF1 protein.

The phrase "separated from a subject" means a state where cells or the like are collected or extracted from a body such that the cells or the like are completely isolated from the body from which the cells or the like are derived. The collecting method or the like for the biological sample is not particularly limited, and known methods can be used.

The "subject" from which the cells or the like are collected or extracted is animals including human. The animals other than human are not particularly limited, and a variety of livestock, poultry, pets, experimental animals, and the like can be targeted. Specific examples thereof include pigs, cattle, horses, sheep, goats, chickens, wild ducks, ostriches, domestic ducks, dogs, cats, rabbits, hamsters, mice, rats, monkeys, and the like. In addition, the subject is not limited to individuals infected with *Aspergillus fumigatus.* It is also possible to target healthy individuals (including individuals who might have been infected with with *Aspergillus fumigatus)* and non-healthy individuals who are immunodeficient due to organ transplantation, anti-cancer agent administration, HIV infection, or the like and susceptible to an opportunistic infection with *Aspergillus fumigatus.*

In the present invention, the "YMAF1 protein" is typically a protein comprising the amino acid sequence of SEQ ID NO: 32. However, the amino acid sequence of a protein may be mutated naturally (i.e., non-artificially) by a mutation or the like in a gene encoding the protein. Thus, the "YMAF1 protein" to be detected in the present invention includes such naturally-occurring mutants. Naturally-occurring mutants normally comprise the aforementioned typical amino acid sequence in which one or more amino acids are substituted, deleted, inserted, or added. Generally, 10 amino acids or less (for example, 5 amino acids or less, 3 amino acids or less, 1 amino acid) in the amino acid sequence are substituted, deleted, inserted, or added. An example of the naturally-occurring mutants includes a protein comprising the amino acid sequence specified by GenBank Accession No: XP_731487.1 (a protein comprising the amino acid sequence of SEQ ID NO: 54). Note that SEQ ID NO: 31 shows a typical example of a base sequence of a gene encoding the protein comprising the amino acid sequence of SEQ ID NO: 32. Moreover, SEQ ID NO: 53 shows a typical example of a base sequence (genome sequence) of a gene encoding the protein comprising the amino acid sequence of SEQ ID NO: 54.

In the present invention, the phrase "detecting a presence of a YMAF1 protein" means to include both detecting whether the YMAF1 protein is present or not, and detecting the degree of the presence of the YMAF1 protein. An amount of the YMAF1 protein present can be grasped as an absolute amount or a relative amount. When the amount present is to be grasped, the amount can be determined, for example, in comparison with an amount of the YMAF1 protein present in a prepared reference sample. The "reference sample" is a sample which has been identified regarding whether or not the YMAF1 protein is expressed in advance. For example, a serum derived from an individual infected with *Aspergillus fumigatus* can be used as the reference sample according to the present invention. In addition, a serum derived from a healthy individual not infected with *Aspergillus fumigatus* can also be used as the reference sample according to the present invention.

Moreover, in "detecting the presence of the YMAF1 protein," *Aspergillus fumigatus* contained in the biological sample is cultured to prepare a culture, which may be used as the target of the detection. An example of the method for preparing such a culture includes, as illustrated in Examples later, a method in which *Aspergillus fumigatus* adhering to or contained in the biological sample is cultured at 25 to 37°C in an *Aspergillus* minimal medium (AMM), an SD medium, a PDA medium, a YPD medium, a Spider medium, a PDA medium, a YG medium, such media supplemented with bovine serum, or the like.

In the present invention, the presence of the YMAF1 protein can be detected by using a substance capable of specifically recognizing and binding to the YMAF1 protein, such as an antibody against the YMAF1 protein, a nucleic acid aptamer for the YMAF1 protein, or the like. Among these, a detection method using an antibody against the YMAF1 protein (immunologicalmethod) is preferable because quick detection is possible with a favorable sensitivity, and because the operation is also easy.

In the immunological method, an antibody against the YMAF1 protein (anti-YMAF1 protein antibody) is used. The antibody is brought into contact with the YMAF1 protein, and the YMAF1 protein is detected on the basis of the binding (bound amount) of the antibody to the YMAF1 protein. Here the term "contact" means that the antibody and the YMAF1 protein are placed under physiological conditions where the anti-YMAF1 protein antibody can recognize the YMAF1 protein.

The "antibody" used in the immunological method may be a polyclonal antibody, a monoclonal antibody, or a functional fragment of the antibodies. Moreover, the "antibody" includes all classes and subclasses of immunoglobulins. The antibody of the present invention is an antibody separated and/or collected (i.e., isolated) from a component in a natural environment. In the present invention, the "functional fragment" of the antibodies means a part of an antibody (partial fragment), which specifically recognizes the target protein. Specific examples thereof include Fab, Fab', F(ab')2, a variable region fragment (Fv), a disulfide bonded Fv, a single chain Fv (scFv), a sc (Fv) 2, a diabody, a polyspecific antibody, polymers thereof, and the like.

When the antibody of the present invention is a polyclonal antibody, the polyclonal antibody can be obtained as follows. Specifically, an animal to be immunized is immunized with an antigen (the target protein, a partial peptide thereof, cells expressing these, or the like). An antiserum from the animal is purified by conventional means (for example, salting-out, centrifugation, dialysis, column chromatography, or the like) to obtain the polyclonal antibody. Meanwhile, a monoclonal antibody can be prepared by a hybridoma method or a recombinant DNA method.

Examples of the immunological method used in the present invention include ELISA, immunohistochemical staining, flow cytometry, radioimmunoassay, immunoprecipitation, western blotting, antibody array, immunochromatography, and the like.

Among the immunological methods, ELISA is preferable from the viewpoint of high specificity and sensitivity.

The ELISA according to the present invention can be performed by those skilled in the art employing known methods as appropriate using the antibody against the YMAF1 protein. For example, in a sandwich ELISA, first, the anti-YMAF1 protein antibody (capture antibody) fixed to a plate is allowed to capture the YMAF1 protein in the biological sample, fragments of *Aspergillus fumigatus* cells containing the YMAF1 protein, or the *Aspergillus fumigatus* cells themselves. Then, an enzyme-labeled anti-YMAF1 protein antibody (detection antibody) to be described later is allowed to act on the captured YMAF1 protein or the like to detect the YMAF1 protein chemically or optically.

In the ELISA according to the present invention, the capture antibody and the detection antibody may be the same or different antibodies from each other, as long as the YMAF1 protein is recognized. From the viewpoint that the YMAF1 protein can be non-competitively captured and detected, the antibodies are preferably different. Examples of combinations of such different antibodies are: the capture antibody is an anti-YMAF1 protein polyclonal antibody, while the detection antibody is an anti-YMAF1 protein monoclonal antibody; the capture antibody is an anti-YMAF1 protein monoclonal antibody, while the detection antibody is an anti-YMAF1 protein polyclonal antibody; and the capture antibody is an anti-YMAF1 protein monoclonal antibody, while the detection antibody is an anti-YMAF1 protein monoclonal antibody capable of recognizing a site (epitope) that is different from a site recognized by the capture antibody.

The antibody of the present invention is preferably an antibody capable of recognizing a region comprising the amino acid sequence at positions 1 to 33 of an extracellular region of the YMAF1 protein (the region comprising the amino acid sequence of SEQ ID NO: 33).

The antibody of the present invention is more preferably: an antibody comprising a light chain variable region including light chain CDR1 to CDR3 and a heavy chain variable region including heavy chain CDR1 to CDR3 of a 4B6M2K antibody, a 1B4C antibody, or a 3G4FB7 antibody to be described in the present Examples; or amino acid sequence mutants thereof. Specifically, the following antibodies are preferable.

"Antibody comprising variable regions including CDRs of 4B6M2K antibody":
(a) an antibody capable of binding to the YMAF1 protein, and comprising
   a light chain variable region including amino acid sequences of SEQ ID NOs: 1 to 3 or the amino acid sequences in at least any one of which one or more amino acids are substituted, deleted, added, and/or inserted, and
   a heavy chain variable region including amino acid sequences of SEQ ID NOs: 4 to 6 or the amino acid sequences in at least any one of which one or more amino acids are substituted, deleted, added, and/or inserted.

"Antibody comprising variable regions including CDRs of 1B4C antibody":
(b) an antibody capable of binding to the YMAF1 protein, and comprising
   a light chain variable region including amino acid sequences of SEQ ID NOs: 7 to 9 or the amino acid sequences in at least any one of which one or more amino acids are substituted, deleted, added, and/or inserted, and
   a heavy chain variable region including amino acid sequences of SEQ ID NOs: 10 to 12 or the amino acid sequences in at least any one of which one or more amino acids are substituted, deleted, added, and/or inserted.

"Antibody comprising variable regions including CDRs of 3G4FB7 antibody":
(c) an antibody capable of binding to the YMAF1 protein, and comprising
   a light chain variable region including amino acid sequences of SEQ ID NOs: 13 to 15 or the amino acid sequences in at least any one of which one or more amino acids are substituted, deleted, added, and/or inserted, and
   a heavy chain variable region including amino acid sequences of SEQ ID NOs: 16 to 18 or the amino acid sequences in at least any one of which one or more amino acids are substituted, deleted, added, and/or inserted.

The antibody of the present invention is particularly preferably: an antibody comprising a light chain variable region and a heavy chain variable region of the antibodies described in the present Examples; or the amino acid sequence mutants thereof. Specifically, the following antibodies are preferable.

"Antibody comprising variable regions of 4B6M2K antibody":
(a) an antibody capable of binding to the YMAF1 protein, and comprising
   a light chain variable region including the amino acid sequence of SEQ ID NO: 20, the amino acid sequence from which a signal sequence is removed, or at least any one of these amino acid sequences in which one or more amino acids are substituted, deleted, added, and/or inserted, and
   a heavy chain variable region including the amino acid sequence of SEQ ID NO: 22, the amino acid sequence from which a signal sequence is removed, or at least any one of these amino acid sequences in which one or more amino acids are substituted, deleted, added, and/or inserted.

"Antibody comprising variable regions of 1B4C antibody":
(b) an antibody capable of binding to the YMAF1 protein, and comprising
   a light chain variable region including the amino acid sequence of SEQ ID NO: 24, the amino acid sequence from which a signal sequence is removed, or at least any one of these amino acid sequences in which one or more amino acids are substituted, deleted, added, and/or inserted, and
   a heavy chain variable region including the amino acid sequence of SEQ ID NO: 26, the amino acid sequence from which a signal sequence is removed, or at least any one of these amino acid sequences in which one or more amino acids are substituted, deleted, added, and/or inserted.

"Antibody comprising variable regions of 3G4FB7 antibody":
(c) an antibody capable of binding to the YMAF1 protein, and comprising
   a light chain variable region including the amino acid sequence of SEQ ID NO: 28, the amino acid sequence from which a signal sequence is removed, or at least any one of these amino acid sequences in which one or more amino acids are substituted, deleted, added, and/or inserted, and
   a heavy chain variable region including the amino acid sequence of SEQ ID NO: 30, the amino acid sequence from which a signal sequence is removed, or at least any one of these amino acid sequences in which one or more amino acids are substituted, deleted, added, and/or inserted.

The amino acid sequence mutants of the antibody of the present invention can be prepared by introduction of a mutation into a DNA encoding an antibody chain, or by peptide synthesis. The modified site of the amino acid sequence of the antibody may be a constant region of the heavy chain or light chain of the antibody, or may be a variable region (framework region and CDR) thereof, as long as the resulting antibody has an equivalent activity to that of the antibody before the modification. Presumably, modification of amino acids other than the CDR has a relatively small influence on binding affinity for the antigen. Meanwhile, there are currently known screening methods for antibodies whose affinity for an antigen is enhanced by modification of amino acids in the CDR (PNAS, 102: 8466-8471 (2005), Protein Engineering, Design & Selection, 21: 485-493 (2008), International Publication No. WO2002/051870, J. Biol. Chem., 280: 24880-24887 (2005), Protein Engineering, Design & Selection, 21: 345-351 (2008)).

The number of amino acids modified is preferably 10 amino acids or less, more preferably 5 amino acids or less, and most preferably 3 amino acids or less (for example, 2 amino acids or less, 1 amino acid). The modification of amino acids is preferably conservative substitution. In the present invention, the "conservative substitution" means substitution with another amino acid residue having a chemically similar side chain. Groups of amino acid residues having chemically similar amino acid side chains are well known in the technical field to which the present invention pertains. For example, amino acid residues can be grouped into acidic amino acids (aspartic acid and glutamic acid), basic amino acids (lysine, arginine, histidine), and neutral amino acids. The neutral amino acids can be classified into amino acids having a hydrocarbon chain (glycine, alanine, valine, leucine, isoleucine, proline), amino acids having a hydroxy group (serine, threonine), amino acids containing sulfur (cysteine, methionine), amino acids having an amide group (asparagine, glutamine), an amino acid having an imino group (proline), and amino acids having an aromatic group (phenylalanine, tyrosine, tryptophan). The amino acid sequence mutants preferably have an equivalent antigen-binding activity to that of a target antibody (typically, the antibodies described in the present Examples). The binding activity to the antigen can be evaluated, for example, by preparing Ba/F3 cells expressing the antigen to analyze the reactivity with the antibody sample using a flow cytometer (see Example 3 to be described later). Moreover, the binding activity to the antigen can be evaluated as described above, for example, by western blotting described in Example 3 later.

Once obtaining the antibody described in the present Examples, those skilled in the art can produce various antibodies which bind to a peptide region (epitope) specified on the protein recognized by the antibody. The epitope of the antibody can be determined by well-known methods such as checking binding to an overlapping synthetic oligopeptide obtained from the amino acid sequence of the target protein (for example, Ed Harlow and D. Lane, Using Antibodies, a Laboratory Manual, Cold Spring Harbor Laboratory Press; US Patent No. 4708871). A peptide library in phage display can also be used for the epitope mapping. Whether two antibodies bind to the same epitope or sterically overlapping epitopes can be determined by a competitive assay method.

The above-described antibodies are useful not only in the testing method of the present invention, but also in a method for preventing or treating an *Aspergillus fumigatus* infection to be described later.

When the antibodies are used in the testing method of the present invention, an antibody bound to a labeling substance can be used. By detecting the label, an amount of the antibody bound to the target protein can be measured directly. The labeling substance is not particularly limited, as long as the labeling substance can bind to the antibody and can be detected by a chemical or optical method. Examples thereof include peroxidases, β-D-galactosidase, microperoxidase, horseradish peroxidase (HRP), fluorescein isothiocyanate (FITC), rhodamine isothiocyanate (RITC), alkaline phosphatases, biotin, radioactive substances, and the like.

Further, besides the method for directly measuring the amount of the antibody bound to the target protein using the antibody bound to a labeling substance, it is possible to utilize indirect detection methods such as a method utilizing a secondary antibody bound to a labeling substance and a method utilizing a polymer bound to a secondary antibody and a labeling substance. Here, the "secondary antibody" is an antibody that exhibits specific binding to the antibody of the present invention. For example, when the antibody of the present invention is prepared as a rabbit antibody, an anti-rabbit IgG antibody can be used as the secondary antibody. Labeled secondary antibodies usable to antibodies derived from various species such as rabbits, goats, and mice are commercially available. In the present invention, it is possible to use a secondary antibody selected as appropriate in accordance with the species from which the antibody of the present invention is derived. Protein G, Protein A, or the like, to which a labeling substance is bound can also be used instead of a secondary antibody.

Information obtained by performing the above-described method targeting those other than a patient having an Aspergillus fumigatus infection, that is, those who are not diagnosed as *an Aspergillus fumigatus* infection can be utilized for determination, evaluation, and so forth regarding whether or not an *Aspergillus fumigatus* infection has been developed. On the other hand, information obtained by performing the method targeting a patient having an Aspergillus fumigatus infection can be utilized for evaluation or grasping of the pathological condition of the patient, the evaluation of the therapeutic effect, and so forth. For example, when the method of the present invention is performed together with a treatment for an *Aspergillus fumigatus* infection, the therapeutic effect can be evaluated based on the resulting information thus obtained. Specifically, whether the YMAF1 protein is present or not in a biological sample separated from a patient and a change in the amount of the YMAF1 protein present are examined by performing the method of the present invention after drug administration, and the therapeutic effect can be determined on the basis of a change in increase or decrease of the amount present. In this manner, the method of the present invention may be utilized for monitoring a therapeutic effect.

The testing of an *Aspergillus fumigatus* infection in a subject is normally conducted by a doctor (including one instructed by a doctor. The same shall apply hereinafter). The data related to the presence of the YMAF1 protein in a biological sample, which are obtained by the method of the present invention, are useful for a diagnosis by a doctor. Thus, the method of the present invention can also be stated as a method for collecting and presenting data useful for a diagnosis by a doctor.

### <Composition for Testing Aspergillus Fumigatus Infection>

Moreover, the present invention provides a composition for testing an *Aspergillus fumigatus* infection, comprising the antibody against the YMAF1 protein. The antibody used in the composition for the testing of the present invention may be labeled as described above. The composition for the testing of the present invention may comprise other ingredients acceptable as a composition in addition to the antibody ingredient. Examples of such other ingredients include a carrier, an excipient, a disintegrator, a buffer, an emulsifier, a suspension, a stabilizer, a preservative, an antiseptic, a physiological salt, a labeled compound, a secondary antibody, and the like. As the excipient, lactose, starch, sorbitol, D-mannitol, white sugar, or the like can be used. As the disintegrator, starch, carboxymethyl cellulose, calcium carbonate, or the like can be used. As the buffer, a phosphate, a citrate, an acetate, or the like can be used. As the emulsifier, gumarabic, sodiumalginate, tragacanth, or the like can be used. As the suspension, glyceryl monostearate, aluminium monostearate, methyl cellulose, carboxymethyl cellulose, hydroxymethyl cellulose, sodium lauryl sulfate, or the like can be used. As the stabilizer, propylene glycol, diethylin sulfite, ascorbic acid, or the like can be used. As the preservative, phenol, benzalkonium chloride, benzyl alcohol, chlorobutanol, methylparaben, or the like can be used. As the antiseptic, sodium azide, benzalkonium chloride, para-hydroxybenzoic acid, chlorobutanol, or the like can be used.

Further, in addition to the composition for the testing of the present invention, a substrate necessary for detection of a label, a positive control or a negative control, a buffer solution used to dilute or wash a sample, or the like can be combined so as to provide a kit for testing an *Aspergillus fumigatus* infection. Moreover, in a case where the antibody preparation is an unlabeled antibody, a labeled substance (for example, secondary antibody, Protein G, Protein A, or the like) capable of binding to the antibody can also be combined. Further, such a kit for testing an *Aspergillus fumigatus* infection may comprise an instruction of the kit.

### <Composition and Methods for Preventing and Treating Aspergillus Fumigatus Infection>

As illustrated in Examples later, the present inventors have revealed that the YMAF1 protein is involved in the pathogenicity of *Aspergillus fumigatus,* and that administering the antibody against the protein improves the survival rate of mice having an *Aspergillus fumigatus* infection.

Thus, the present invention provides a composition for preventing or treating an *Aspergillus fumigatus* infection, comprising the antibody against the YMAF1 protein.

The antibody comprised in the composition for preventing or treating an *Aspergillus fumigatus* infection of the present invention includes, in addition to those described above, a chimeric antibody, a humanized antibody, a human antibody, and a functional fragment of these antibodies. Among these, a chimeric antibody, a humanized antibody, or a human antibody is desirable from the viewpoint of side effect reduction.

In the present invention, a "chimeric antibody" is an antibody obtained by linking a variable region of an antibody of one species to a constant region of an antibody of another species. A chimeric antibody can be obtained as follows, for example. Specifically, a mouse is immunized with an antigen. A portion corresponding to an antibody variable part (variable region) which binds to the antigen is cut out from a gene of a monoclonal antibody of the mouse. The portion is linked to a gene of a constant part (constant region) of an antibody derived from human bone marrow. This is incorporated into an expression vector, which is then introduced into a host for the production of a chimeric antibody (for example, Japanese Unexamined Patent Application Publication No. Hei 8-280387, US Patent No. 4816397, US Patent No. 4816567, US Patent No. 5807715). Moreover, in the present invention, a "humanized antibody" is an antibody obtained by grafting (CDR grafting) a gene sequence of an antigen-binding site (CDR) of a non-human-derived antibody onto a human antibody gene. The preparationmethods are known (see, for example, EP239400, EP125023, WO90/07861, WO96/02576). In the present invention, a "human antibody" is an antibody, all regions of which are derived from human. In preparing a human antibody, it is possible to utilize a transgenic animal (for example, a mouse) capable of producing a repertoire of the human antibody by immunization. Preparation methods for a human antibody are known (for example, Nature, 1993, 362, 255-258, Intern. Rev. Immunol, 1995, 13, 65-93, J. Mol. Biol, 1991, 222, 581-597, Nature Genetics, 1997, 15, 146-156, Proc. Natl. Acad. Sci. USA, 2000, 97: 722-727, Japanese Unexamined Patent Application Publication No. Hei 10-146194, Japanese Unexamined Patent Application Publication No. Hei 10-155492, Japanese Patent No. 2938569, Japanese Unexamined Patent Application Publication No. Hei 11-206387, International Application Japanese-Phase Publication No. Hei 8-509612, International Application Japanese-Phase Publication No. Hei 11-505107).

The composition for the prevention and treatment of the present invention can be formulated by known formulation methods. The composition can be used orally or parenterally in the form of, for example, a capsule, a tablet, a pill, a liquid, a powder, a granule, a fine granule, a film coating agent, a pellet, a troche, a sublingual tablet, a masticatory, a buccal, a paste, a syrup, a suspension, an elixir, an emulsion, a topical agent, an ointment, a plaster, a poultice, a percutaneous absorption preparation, a lotion, an inhalation, an aerosol, an injection, a suppository, or the like.

When formulated, these can be combined as appropriate with a carrier acceptable pharmacologically or as a food or drink, specifically, sterile water, a saline, a vegetable oil, a solvent, a base, an emulsifier, a suspension, a surfactant, a stabilizer, a flavor, an aromatic, an excipient, a vehicle, an antiseptic, a binder, a diluent, an isotonic agent, a soothing agent, a filler, a disintegrator, a buffer, a coating agent, a lubricant, a colorant, a sweetener, a viscous agent, a corrigent, a solubilizer, or other additives.

The composition for the prevention and treatment of the present invention may be used in combination with a known composition used for preventing or treating an *Aspergillus fumigatus* infection. Examples of such a known composition include an azole antifungal drug and an echinocandin antifungal drug. Alternatively, the composition for the prevention and treatment of the present invention may be used in combination with a drug (for example, immunosuppressant, anti-cancer agent, HIV treatment drug used during or after organ transplantation, or other timing) for patients who are susceptible to an opportunistic infection with *Aspergillus fumigatus.*

When the composition for the prevention and treatment of the present invention is administered, the amount administered is selected as appropriate in accordance with the age, weight, symptom, and health state of the target, the type of the composition, and so forth. For example, the amount of the composition for the prevention and treatment of the present invention administered at one time is generally 0.01 mg/kg body weight to 100 mg/kg body weight.

As described above, the present invention makes it possible to prevent or treat an *Aspergillus fumigatus* infection by administering the composition of the present invention to a patient having an Aspergillus fumigatus infection or a patient at a risk of infection with *Aspergillus fumigatus.* Thus, the present invention also makes it possible to provide a method for preventing or treating an *Aspergillus fumigatus* infection, comprising a step of administering the antibody against the YMAF1 protein.

A product (drug) of the composition for the prevention and treatment of the present invention or a protocol thereof may be labeled to indicate that the use is to prevent or treat an *Aspergillus fumigatus* infection. Herein, the phrase "a product or a protocol is labeled" means that the body of the product, a container or a package therefor, or the like is labeled, or that a protocol, an attachment document, an advertisement, other prints, or the like disclosing information on the product is labeled.

### <Screening Method for Compound for Preventing, Treating, and Testing Aspergillus fumigatus Infection>

Furthermore, the present invention also provides a screening method for a compound for testing, preventing, or treating an *Aspergillus fumigatus* infection, the method comprising the steps of:
bringing a test compound into contact with any one of a YMAF1 protein and a portion thereof; and
selecting a compound bound to any one of the YMAF1 protein and the portion thereof.

The "test compound" used in the screening method of the present invention is not particularly limited. Examples thereof include an expression product from a gene library, a synthetic low-molecular-weight compound library, a peptide library, an antibody, a substance released by a bacterium, a liquid extract and a culture supernatant of cells (microorganisms, plant cells, animal cells), a purified or partially purified polypeptide, an extract derived from a marine organism, plant, or animal, soil, and a random phage peptide display library.

Moreover, examples of the "YMAF1 protein" used here include the protein comprising the amino acid sequence of SEQ ID NO: 32, the protein comprising the amino acid sequence of SEQ ID NO: 54, and the protein comprising the amino acid sequence specified by GenBank Accession No: XP_731487.1. Further, the portion of the YMAF1 protein is not particularly limited, but is preferably a polypeptide comprising the amino acid sequence at positions 1 to 33 of the extracellular region of the YMAF1 protein (for example, a polypeptide comprising the amino acid sequence of SEQ ID NO: 33).

The YMAF1 protein or the portion thereof can also be used in the form of a fusion protein with another protein for facilitating the detection (for example, an enzyme such as alkaline phosphatase (SEAP) and β-galactosidase, a glutathione S-transferase (GST), or a fluorescent protein such as a green fluorescent protein (GFP)), as necessary.

The YMAF1 protein or the portion thereof may be used in the form of a purified protein, or may be used in the form of a protein expressed in a cell or the like.

The test compound can be brought into contact with the YMAF1 protein or the portion thereof, for example, by adding the test compound to a system containing the purified protein or by adding the test compound to a culture solution in which the cells expressing the protein are cultured. Moreover, the binding between the test compound and the YMAF1 protein or the portion thereof can be detected by known methods, for example, co-immunoprecipitation, yeast two-hybrid system, ELISA, a method using a detection system utilizing surface plasmon resonance (for example, BIAcore (manufactured by GE Healthcare)), and a method utilizing FRET (fluorescence resonance energy transfer).

In screening for the compound for preventing or treating an *Aspergillus fumigatus* infection, in addition to the steps (a) and (b), it is possible to further perform a step of analyzing whether or not the compound bound to the YMAF1 protein or the portion thereof selected in the step (b) has an activity of suppressing the pathogenicity of *Aspergillus fumigatus.* An example of the method for analyzing whether or not to have an activity of suppressing the pathogenicity of *Aspergillus fumigatus* includes, as illustrated in Examples later, a method in which when *Aspergillus fumigatus* is cultured in a medium having the compound added thereto, whether or not the *Aspergillus fumigatus* has reduced aggregation and spore-forming ability is analyzed in comparison with culturing in a medium to which the compound is not added (see Example 4). Moreover, the example includes a method in which the compound is administered to experimental mice having aspergillosis (invasive *Aspergillus* model mice), and whether or not the survival rate is improved is analyzed in comparison with a case of not administering the compound (see Example 6).

### [Examples]

Hereinafter, the present invention will be described more specifically based on Examples. However, the present invention is not limited to the following Examples.

### (Example 1) Executing SST-REX

SST-REX was executed to comprehensively obtain information on a gene encoding a membrane protein or a secretory protein expressed on the cell surface of *Aspergillus fumigatus.*

### (1) Preparation of cDNAs

Conidia of a clinically isolated strain MF-13 of *Aspergillus fumigatus* were cultured in a YPD medium at 37°C for 3 days. Mycelia were formed from the conidia by the culturing, and the mycelia were further grown into a filamentous form with a diameter of approximately 5 to 10 mm. Then, after *Aspergillus fumigatus* was collected, total RNA was prepared from the fungus. Subsequently, 12 µg of mRNAs were obtained from the total RNA as the material using FastTrack2.0 mRNA Isolation kit (manufactured by Invitrogen Corp., #K1593-02). Thereafter, using SuperScript (TM) Choice System (manufactured by Invitrogen Corp., #18090-019), double-stranded cDNAs were prepared from 3 µg of the obtained mRNAs.

### (2) Incorporation (Chimerization) of cDNA Sequenc e into pMX-SST Vector

To incorporate the obtained cDNAs into a retrovirus vector pMX-SST, 5 µg of the pMX-SST vector (see Kojima T. And Kitamura T., Nature Biotechnology, 1999, vol. 17, pp. 487 to 490) was subjected to a cleavage treatment using a restriction enzyme BstXI in 100 µl of a reaction system at 45°C for 4 hours. All the reaction solution was electrophoresed on a 1% agarose gel, and a DNA fragment of approximately 5000 bases in length corresponding to the vector site was cut out. Further, using Wizard(R) SV Gel and PCR Clean-Up System (manufactured by Promega Corporation, #A9282), the DNA fragment of approximately 5000 bases in length was purified. The DNA fragment thus obtained was of the pMX-SST vector treated with the BstXI restriction enzyme, and an aqueous solution containing 50 ng of the DNA fragment per µl was prepared.

The double-stranded cDNA prepared above has blunt ends, and cannot be directly ligated to the pMX-SST treated with the BstXI restriction enzyme. For this reason, an operation was performed, so that both ends of the double-stranded cDNA had a DNA sequence obtained after the cleavage with the BstXI restriction enzyme. Nine µg of a BstXI adapter (manufactured by Invitrogen Corp., #N408-18) was dissolved in 10 µl of water, and the double-stranded cDNAs were further dissolved in the BstXI adapter aqueous solution. To this, 5 µl of LigationHigh (manufactured by TOYOBO Co. , Ltd., #LGK-201) was added and suspended for reaction at 16°C for 16 hours. Thereby, the BstXI adapter and the double-stranded cDNAs were ligated. Thereafter, the resulting DNAs prepared in the above described manner were each electrophoresed on a 1.5% agarose gel. After that, the gels containing the ligated products of the BstXI adapter and the double-stranded cDNA fragments having a length from approximately 500 bases to approximately 4000 bases were cut out. Using Wizard(R) SV Gel and PCR Clean-Up System, the ligated products of the double-stranded cDNAs and the BstXI adapter were purified.

Then, 50 ng of the pMX-SST vector treated with the BstXI restriction enzyme, a total amount of the resulting ligated products of the double-stranded cDNAs and the BstXI adapter purified above, and a T4 DNA ligase were treated in 20 µl of a reaction system at room temperature for 3 hours. The pMX-SST vector treated with the BstXI restriction enzyme was ligated to the ligated products. Note that the composition of the reaction solution was adjusted according to the specification.

### (3) Amplification of cDNA Libraries

The cDNA libraries constructed using the pMX-SST vector were introduced and amplified in *Escherichia coli.* To the cDNA libraries, 5 µg of tRNA, 12.5 µl of 7.5 M sodium acetate, and 70 µl of ethanol were added, mixed by inverting, followed by centrifugation at 20,400 × g for 30 minutes. The supernatant was discarded, and a precipitate was obtained. To the obtained precipitate, 500 µl of 70% ethanol was added, followed by centrifugation at 20,400 × g for 5 minutes. A precipitate obtained by discarding the supernatant was dissolved in 6 µl of water. To amplify the cDNAs in *Escherichia coli,* 2 µl of the solution was mixed with 23 µl of competent cells (manufactured by Invitrogen Corp., #18920-015), followed by electroporation under a condition of 1.8 kV. A total amount of the resultant was suspended in 1 ml of an SOC medium. This operation was performed twice. The SOC medium, in which *Escherichia coli* was suspended, was subjected to shaking culture at 37 °C for 90 minutes. Thereafter, a total amount of this culture solution was inputted into 300 ml of an LB medium containing 100 µg of ampicillin per ml of the medium, followed by shaking culture at 37°C for 16 hours. Note that the composition of the LB medium included tryptone 1% (w/v), yeast extract 0.5% (w/v), and sodium chloride 1% (w/v).

Meanwhile, to check the number of the cDNA libraries introduced in *Escherichia coli* and the chain length of the cDNAs ligated to the pMX-SST vector, 3 µl of the culture solution was taken out and plated on an LB agar medium containing 50 µg/ml of ampicillin. As a result, growth of 280 colonies was observed on the 3 µl-plated LB agar medium. This suggested that there were 2.8 × 10⁷ independent cDNA libraries in 500 ml of the culture solution.

Moreover, plasmids were extracted from certain 16 of the colonies, and subjected to a restriction enzyme treatment with the BstXI restriction enzyme. The treated products were each electrophoresed on a 1% agarose gel, and the length of the cDNAs on the pMX-SST vector was measured. As a result, an average value thereof was approximately 1200 bases.

Furthermore, plasmids collected from the remaining culture solution were purified using NucleoBond (R) AX 500 columns (manufactured by NIPPON Genetics Co., Ltd., #740574), and an amplified cDNA library system was established.

### (4) Packaging of cDNA Libraries and Executing SST-REX method

To produce a retrovirus containing a pMX-SST retrovirus vector RNA, in which a cDNA library-derived gene was incorporated, 2 × 10⁶ virus packaging cells Plat-E (see Morita S. et al., Gene Ther., 2000 June, vol. 7, no. 12, pp. 1063 to 1066) were suspended in a 6-cm dish containing 4 ml of a DMEM medium (manufactured by Wako Pure Chemical Industries, Ltd., #044-29765), and cultured under conditions of 37°C and 5% CO₂ for 24 hours. On the other hand, 100 µl of opti-MEM (manufactured by GIBCO, #31985070) and 9 µl of Fugene (manufactured by Roche Applied Science, #1814443) were mixed and left standing for 5 minutes at room temperature. Then, 3 µg of the cDNA libraries were added thereto and left standing for 15 minutes at room temperature. The solution containing the cDNA libraries was added dropwise to the cultured Plat-E cells. After 24 hours, the supernatant was replaced, and the culturing was continued under the same conditions. The supernatant after another 24 hours was filtered through a 0.45-µm filter.

Into a 10-cm dish having 9.5 ml of an RPMI-1640 medium (manufactured by Kohjin Bio Co., Ltd.) containing 4 × 10⁶ Ba/F3 cells, 0.5 ml of the filtered supernatant thus obtained was added.

Further, 10 µl of polybrene (manufactured by CHEMICON, #TR-1003-G) and 10 ng of IL-3 were added, followed by culturing for 24 hours. Then, the cells were washed with an RPMI-1640 medium three times, and suspended in 200 ml of a fresh RPMI-1640 medium. The cells were spread in an equal amount on each of twenty 96-well plates. Selection and cloning were attempted based on the autonomous replication ability of the Ba/F3 cells. Cells whose growth was observed after 10 days to 20 days were selected based on SST-REX, and culturing was further continued until the cells grew all over the wells.

### (5) Analysis of Gene Product Obtained by SST-REX

Half the amount of the cells obtained from each well was cultured to expand as cell stocks. Further, the cells from the cell stocks were cultured. Transfectant Ba/F3 cells extracellularly expressing peptide molecules derived from the incorporated cDNAs were used as immunogen cells for preparing an antibody, and as screening target cell. From the remaining half of the cells obtained from each well, the genome was extracted, followed by sequencing to analyze genes derived from the introduced cDNAs. In the sequencing, PCR was performed on the obtained genome using PrimeSTAR MAX DNA polymerase (manufactured by Takara Bio Inc. , #R045A). Note that primers having the following sequences were used in the PCR.
SST3' side-T7 5'-TAATACGACTCACTATAGGGCGCGCAGCTGTAAACGG TAG-3' (SEQ ID NO: 34)
SST5' side-T3 5'-ATTAACCCTCACTAAAGGGAGGGGGTGGACCATCCTC TA-3' (SEQ ID NO: 35).

Then, the obtained PCR products were purified using Wizard(R) SV Gel and PCR Clean-Up System and so forth. Then, the purified PCR products were sequenced using BigDye Terminator v3.1 Cycle sequencing (manufactured by ABI, #4337456) and DNA sequencer ABI3100XL. Note that the following was used as a primer in the sequencing.
SST5' side-T3 5'-ATTAACCCTCACTAAAGGGAGGGGGTGGACCATCCTC TA-3' (SEQ ID NO: 35).

The obtained sequence data was analyzed using a BLAST search (http://www.ncbi.nlm.nih.gov/BLAST/) and SignalP 3.0 Server (http://www.cbs.dtu.dk/services/SignalP/).

As a result of executing the SST-REX method using the cells as the material as described above, by sequencing the cDNA-derived genes from 407 cells of the transfectant Ba/F3, 75 different genes were obtained. The transfectant Ba/F3 cell system subjected to the gene analysis was verified to contain only one gene derived from the cDNAs, and used for the subsequent experiments. Hereinafter, the cells containing the cDNA-derived gene thus obtained are referred to as "SST clone cells."

### (Example 2) Cloning of YMAF1 Gene and Construction of Expression System

### (1) Identification and Cloning of Expressed Gene

Genes corresponding to the genes obtained by the SST-REX method in Example 1 and believed to encode a secretory protein or a membrane protein were identified from annotation information described in the genome database of *Aspergillus fumigatus,* and so forth. The functions of many of the identified genes were unknown from the information in the database. Nevertheless, in consideration of the number of the SST clones containing the genes thus obtained, targeted was a gene YMAF1 (YPD medium associated major antigen of *Aspergillus fumigatus,* SST clone cell code: ACT073-502), which was shared by the largest number of the SST clone cells containing the gene, and which was believed to have a high level of expression.

The YMAF1 gene is a gene encoding a conserved hypothetical protein having a molecular weight of approximately 23 KDa based on the database. According to the homology search, a gene exists in *A. clavatus,* which has a homology of 60% with this gene, but no gene having a high homology therewith exists in *A. flavus, A. niger,* and *A. nidulans.*

Next, using oligo-dT of *Aspergillus fumigatus* mRNA as a template, a 1st strand cDNA was synthesized with a reverse transcriptase, and a coding region of the YMAF1 gene was amplified by PCR and cloned in pBluescript II.

Note that the obtained YMAF1 gene and a protein encoded by the gene were shown to differ from a conserved hypothetical protein of *Aspergillus fumigatus* AF293 (AFUA_6G00690), a partial mRNA thereof (GenBank Accession No: XM_726394.1), and a conserved hypothetical protein [*Aspergillus fumigatus* Af293] (GenBank Accession No: XP_731487.1) in base sequence by three positions and in amino acid sequence by one position. The remaining sequences were the same (see Fig. 1).

### (2) Preparation of Recombinant Protein in Budding Yeast (Saccharomyces cerevisiae, S. cerevisiae) Expression System

The cloned YMAF1 gene was inserted in a pADH-HA expression vector configured to add a HA tag to the C-terminus of a protein, and then introduced into *S. cerevisiae.* After a culture supernatant of *S. cerevisiae* thus prepared was collected, the supernatant was immunoprecipitated with an anti-HA antibody, and the resultant was subjected to western blotting for detection of a secretory protein using an anti-HA-antibody. As a result, a band of approximately 23 KDa was observed (see Fig. 2).

### (3) Preparation of Recombinant Protein in Escherichia coli

The region encoding the YMAF1 gene was cloned in a pGEX-6P-1-His6a-Flag vector, expressed in *Escherichia coli,* and cultured in a large amount. The cultured bacterium thus obtained was suspended in a buffer (50 mM Tris-HCl pH 7.5, 100 mM NaCl, 10% glycerol), and subjected to a disruption treatment by ultrasonication to thereby obtain a soluble fraction. Then, a fusion protein between YMAF1 and GST was purified from the soluble fraction thus prepared using a glutathione sepharose column. Subsequently, the recombinant protein thus purified was used for polyclonal antibody production in rabbits, and used as a control of western blotting and sandwich ELISA. Meanwhile, an insoluble fraction in the disruption treatment by ultrasonication was treated with 8 M urea, and the resulting soluble fraction was also collected. Note that, in this *Escherichia coli* expression system, the expression of the YMAF1-GST fusion protein having a molecular weight of approximately 60 KDa was observed in any of the soluble fraction and the insoluble fraction (the 8 M urea soluble fraction) in the disruption treatment by ultrasonication (see Figs. 3 and 4).

### (Example 3) Preparation of YMAF1 Antibody

### (1) Polyclonal Antibody

The vector described in Example 2 (3), in which the YMAF1 gene was cloned, was introduced into *Escherichia coli* BL21, and the recombinant protein was excessively expressed, followed by purification. Specifically, 100 mL of an LB medium was put into a 1-L Erlenmeyer flask, and 1/100 of the culture solution cultured above was further added, followed by shaking culture at 37°C. Then, when O. D. 600 = 0.7, IPTG was put into the culture solution to a final concentration of 1 mM, and the mixture was further shake-cultured at 37°C for 3 hours. Subsequently, approximately 2 mL of a Tris-HCl buffer (pH 7.5) was added to the resulting *Escherichia coli* cells, and sonication was performed on ice to prevent over-heating. Then, the resulting pellets were washed with a Tris-HCl buffer of the same formula as above, and subjected to sonication again. This operation was repeated three times to concentrate the recombinant protein. Thereafter, the concentrated protein solution was separated by SDS-PAGE. The recombinant protein portion was cut out from the gel and disrupted, then immersed in a PBS buffer, electrified at 100 V for a day and a night, and thus eluted from the gel. Then, the eluted protein was concentrated with Amicon Ultra (manufactured by Millipore Corporation, catalog number: UFC901096) and adjusted to 1 mg/mL for use as an immunogen protein.

SPF Japanese white rabbits were used as animals to be immunized. An immunostimulant TiterMax Gold (manufactured by Alexis Biochemicals, ALX-510-002-L010), 100 µL, was mixed with an equivalent amount, 100 µL, of the YMAF1 protein solution (1 mg/mL). The immunogen obtained by emulsification was subcutaneously administered by injection each in an amount of 200 µL per individual, once every other week, 6 times in total for the immunization. After the immunization, the blood was collected from the rabbit, and the serum was collecting using a centrifuge.

Moreover, 10 mg of a GST protein was bound to 3 ml of an activated CNBr-agarose column to prepare an absorption column for an anti-GST antibody contained in the serum. Then, the collected serum was added to the column, and circulated overnight using a perista pump. On the next day, the GST column was washed, and an anti-GST antibody was eluted. Further, the operation was repeated three times on the column-through serum, and a serum with the anti-GST antibody having been adsorbed (removed) (anti-GST antibody-removed serum) was obtained. Note that by reacting the serum with a GST-immobilized ELISA plate, it was confirmed that the anti-GST antibody had lost the activity.

Next, the anti-GST antibody-removed serum was purified using Protein A Sepharose (manufactured by GE Healthcare, 17-1279-03), MAPS-II Binding Buffer (manufactured by Bio-Rad Laboratories, Inc., 153-6161), and a 1 M L-Arg elution buffer. Then, the eluted rabbit IgG was dialyzed with PBS, and a purified antibody fraction (hereinafter, may also be referred to as "anti-YMAF1 polyclonal antibody") was obtained. Moreover, a reactivity test was conducted on the obtained purified antibody fraction using an ELISAplate on which a GST protein was immobilized and an ELISA plate on which a YMAF1 protein was immobilized. It was confirmed that a reaction specific to the YMAF1 protein-immobilized ELISA plate was shown.

Note that the YMAF1 protein-immobilized ELISA plate was prepared as followed. Specifically, 50 mL of the YMAF1 protein solution diluted with PBS to a concentration of 5 µg/mL was added to Maxisorp (manufactured by NUNC, 984688), and left alone at 4°C overnight. On the next day, the reaction solution was discarded, and a PBS solution containing 4% BSA and 5% sucrose was added. The resultant was further left alone at 4°C overnight. On the next day, the reaction solution was discarded, and the resultant was dried in a draft.

Moreover, as to the ELISA reaction, primary antibodies prepared by dilution with PBS at 10 µg/mL, 1 µg/mL, and 0.1 µg/mL were added, by 50 µL/well, to each ELISA plate on which the protein was immobilized. Then, after reaction for 1 hour at room temperature, the resultant was washed with PBS containing 0.05% Tween 20, and an enzyme-labeled secondary antibody (MBL 458) was added for further reaction at room temperature for 1 hour. After the reaction, the resultant was washed with PBS containing 0.05% Tween 20, and a TMB enzyme substrate was added. After 20 minutes, the reaction was ceased with a 1. 5 N phosphoric acid solution. The absorbance at A450 nm was measured with a plate reader.

### (2) Monoclonal Antibody

As an animal to be immunized, a mouse BALB/c was used. First, as an immunostimulant, TiterMax Gold was mixed with an equivalent amount of PBS and emulsified. To the Balb/c mouse, 50 µl of the resultant was administered. On the next day, 5 × 10⁶ SST clone cells (ACT073-502) having the antigen gene were administered thereto as immunogen cells. Further, the immunogen cells were injected 4 times at intervals of 2 days. Approximately 2 weeks after the first immunization, secondary lymphoid tissues were extracted and ground to obtain a cell population including antibody-producing cells. These cells were mixed with fusion partner cells for cell fusion using polyethylene glycol (manufactured by MERCK KGaA, 1.09727.0100). Thereby, hybridomas were prepared. Note that mouse myeloma cells P3U1 (P3-X63-Ag8.U1) were used as the fusion partner cells.

The prepared hybridomas were each cultured for 10 to 14 days inanRPMI 1640 (manufactured by Mako Pure Chemical Industries, Ltd.) selective medium containing a selective medium HAT (manufactured by SIGMA-ALDRICH CO., H0262), 5% BM-condimed (manufactured by Roche Applied Science, 663573),15% FBS, and a 1% penicillin/streptomycin solution (manufactured by GIBCO, 15140-122, Penicillin-streptomycin liquid, hereinafter abbreviated as "P/S"). Next, flow cytometry was performed as primary screening to thereby select hybridomas, which reacted with the immunogen cells ACT073-502 but not with SST clone cells not containing the antigen gene used as negative control cells. The hybridomas were cultured to expand in D-MEM (manufactured by Invitrogen Corp., 802931) selective medium containing HT (manufactured by SIGMA-ALDRICH CO., H0137), 15% FBS containing 30 ml of a culture supernatant of T-24 cells, and P/S at a final concentration 100 units/ml. Then, flow cytometry was performed again as secondary screening to thus select hybridomas, which reacted with the ACT073-502 cells but not with the other Ba/F3-derived cells (negative control) (see Figs. 5 to 8).

As a result, four clones of 1B4C, 2G11GB5, 3G4FB7, and 4B6M2GK were obtained. After these were monocloned, isotypes of the antibody were determined using Iso Strip Kit (manufactured by Roche Applied Science, 1493027). Specifically, the isotype of the 1B4C antibody was IgM, the isotype of the 2G11GB5 antibody was IgG3/κ, the isotype of the 3G4FB7 antibody was IgG3/κ, and the isotype of the4B6M2GK antibody was IgG1/κ.

Note that when a purified antibody was to be obtained from the hybridoma of each monoclonal antibody thus obtained, the hybridoma was acclimatized to a serum-free medium (Hybridoma-SFM: manufactured by GIBCO, 12045-076) and cultured to expand. After culturing for a certain period, a culture supernatant was obtained. IgG fractions contained in the culture supernatant were purified using Protein A Sepharose (manufactured by GE Healthcare, 17-1279-03), MAPS-II Binding Buffer (manufactured by Bio-Rad Laboratories, Inc., 153-6161), and a 1 M L-Arg elution buffer. The eluted IgG was dialyzed with PBS, and a purified antibody fraction was obtained. In the case of 1B4C that was IgM, the purification was performed using MEP Hypercel (manufactured by Biosepra S.A.), acetic acid, and sodium acetate. Then, the eluted IgGs were dialyzed with PBS, and purified antibody fractions were obtained.

Moreover, each of the antibodies was confirmed to have specificity and binding to the YMAF1 protein by western blotting using the recombinant protein prepared in Example 2 (3) (see Fig. 9).

### (Example 4) Analysis of YMAF1 Gene Function

To analyze the function of the protein encoded by the YMAF1 gene, YMAF1 gene-disrupted strains of *Aspergillus fumigatus* and complementation strains thereof were prepared based on constructs shown in Fig. 10. Note that, in preparing these strains, Afs35 derived from a clinically isolated strain D141 was purchased for use from the Fungal Genetics Stock Center. The akuA gene is deleted in this strain, and homologous recombination occurs at a high frequency.

### (1) Preparation of YMAF1 Gene-Disrupted Strains

As to a DNA fragment used to disrupt the YMAF1 gene (DNA fragment for disrupting the YMAF1 gene), first, a genomic DNA of the Afs35 strain was purified and used as a template to amplify approximately 500 bp of a non-coding region on the 5' side of the YMAF1 gene and approximately 500 bp of a non-coding region on the 3' side by PCR. The two were linked to the respective sides of a drug resistance gene (hygromycin-thymidine kinase fusion protein), and cloned in pBluescript II. Then, after confirmed to be an expected recombinant by sequencing, the obtained plasmid was amplified by PCR using itself as a template to thus prepare the DNA fragment for disrupting the YMAF1 gene.

After the Afs35 strain was cultured, the cell wall was digested by an enzyme treatment to prepare the protoplast. Then, the DNA fragment for disrupting the YMAF1 gene was introduced into the protoplast using CaCl₂ and PEG, and the resultant was seeded in an agar medium for selection with hygromycin 200 µg/ml. Subsequently, colonies, which appeared by culturing at 30°C, were separated. After that, spores therefrom were subjected directly to PCR to identify gene-disrupted strains.

As a result, six strains among nine strains analyzed after separation with selective media were successfully obtained as the YMAF1 gene-disrupted strains (d-YMAF1 strains). Note that the reason why a little less than 70 percent of the strains were obtained as homologous recombinants in this manner quite efficiently is presumably because the Ku70 protein of the akuA gene product of the Afs35 strain used as the parental strain is deficient.

### (2) Preparation of YMAF1 Gene Complementation Strains

Using the YMAF1 gene-disrupted strain clones d-YMAF1, YMAF1 gene complementation strains (YMAF1-comp) were prepared. Specifically, first, a plasmid pCR4-YMAF1comp-3HA-ptrA was prepared, in which a 3 x HA peptide tag was added to a 5' region containing a promoter of the YMAF1 gene and to the C-terminus of the YMAF1 gene, and a pyrithiamine resistance gene was linked downstream thereof. Then, using this plasmid as a template, a DNA fragment used for gene introduction was prepared by PCR. The DNA fragment was introduced into the Afs35 strain using CaCl₂ and PEG. The resultant was seeded in an agar medium, and gene introduced strains were obtained by selection with 0.2 µg/ml of pyrithiamine. Moreover, complementation strains were identified by RT-PCR and Southern hybridization from the obtained strains. Figs. 11 and 12 show the obtained result.

Note that, in the Southern hybridization, genomic DNAs were prepared from the parental strain Afs35, the gene deficient strains, and the gene complementation strains by using a DNeasy Plant Mini kit (manufactured by Qiagen GmbH). The genomic DNAs were digested with a restriction enzyme BamHI, and each electrophoresed on a 1% agarose gel for separation, followed by Southern blot. Additionally, probes ("probe A" and "probe Hph" shown in Fig. 10) used were labeled with AlkPhos direct labeling kit and CDP-Star reagent (manufactured by GE Healthcare).

In the detection of YMAF1 mRNAs by the RT-PCR, first, total RNAs were prepared from the parental strain Afs35, the gene deficient strains, and the gene complementation strains using an RNAeasy Mini kit (manufactured by Qiagen GmbH). Then, the prepared total RNAs were reverse transcribed with ReverTra Ace (manufactured by TOYOBO CO., LTD.). Using each of the obtained cDNA fragments as a template, PCR was performed with TaKaRa Ex Taq (manufactured by Takara Bio Inc.). The resultant was electrophoresed on a 2% agarose gel for separation. After that, DNAs amplified with ethidium bromide were detected.

As shown in Fig. 11, the analysis by the Southern hybridization and RT-PCR revealed that two strains were expected complementation strains.

Furthermore, the fungal cells were cultured at 37°C in minimal media (AMM), and RNAs were prepared to examine the YMAF1 gene expression by RT-PCR. As a result, as shown in Fig. 12, no YMAF1 mRNA was detected in a YMAF1 gene-disrupted strain (one of the six d-YMAF1 strains: d-YMAF1-7) as expected, but the YMAF1 expression was observed in the parental strain Afs35 and a YMAF1 gene complementation strain (one of the two YMAF1-comp strains: YMAF1-comp-4).

### (3) Comparison of Growths in Various Media

First, prepared were: an *Aspergillus* minimal (AMM) medium, an SD medium, a PDA medium, a YPD medium, a Spider medium, a YG medium, and agar media obtained by adding fetal bovine serum to these media by 10%.

Incidentally, as to the AMM medium, see R. W. Barratt et al., Genetics, 1965, vol. 52, pp. 233 to 246. Moreover, regarding the AMM medium, media with different carbon sources were prepared and used in culturing to be described later. Specifically, as shown in Fig. 13, prepared were: a 1% glucose-containing AMM medium (AMM + glucose), a 2% sucrose-containing AMM medium (AMM + sucrose), a 2% sorbitol-containing AMM medium (AMM + sorbitol), a 2% glycerol-containing AMM medium (AMM + glycerol), and a 0.2% BSA-containing AMM medium (AMM + BSA). The composition of the SD medium included yeast nitrogen base medium (w/o amino acids)) 0.67%, glucose 2%, and 20 to 50 µg/ml of supplement amino acids and pyrimidines. The PDA medium was prepared by adding 20 g of glucose and 15 g of agar to potato broth (200 to 400 g/L). The composition of the YPD medium included yeast extract 1%, peptone 2%, and glucose 2%. As to the Spider medium, see H Liu et al., Science, 1994, vol. 266, no. 5191, pp. 1723 to 1726. As to the YG medium, see Edyta Szewczyk1 et al., nature Protocols, 2007, vol. 1, pp. 3111 to 3120.

Subsequently, spore solutions of the d-YMAF1 and the YMAF1-comp were spotted on these media, and cultured for 3 days at 25°C, 30°C, or 37°C. Growths thereof were compared with that of the parental strain Afs35. Figs. 13 to 15 show the obtained result. Moreover, Figs. 16 and 17 show the result of observing the growth state and the conidium state in the Spider medium supplemented with bovine serum by 10%.

As shown in Figs. 13 and 14, no difference was observed in the growth rate among the various media. Note that, although unillustrated, in the cases of 25°C and 37°C also, there was no difference in the *Aspergillus* growth state as in the case of 30°C shown in Fig. 13.

Nevertheless, as shown in Fig. 15, a difference in the state of the colony surface was observed only in the Spider medium containing 10% fetal bovine serum (FBS). Note that there was no difference in the form of conidial heads as shown in Fig. 16.

Moreover, as shown in Fig. 17, when grown at 25°C in the PDA medium supplemented with 10% serum, the spore of the YMAF1 gene-deficient (disrupt) strain was whitish, and the state of the spore formation was also poor. This suggested that the YMAF1 gene-deficient strain had a reduced spore-forming ability.

Next, to confirm that if the YMAF1 protein was deficient, this made a difference in the spore-forming ability, the parental strain Afs35, the YMAF1 gene-deficient strain, and the YMAF1 gene complementation strain were cultured in minimal media (AMM) at 25°C, 30°C, or 37°C. Then, spores were washed off with 0.05% Tween80 PBS, and the number of spores was counted with a hemocytometer. Fig. 18 shows the obtained result.

As apparent from the result shown in Fig. 18, it was revealed that when grown at 25°C in the PDA medium, the YMAF1 gene-deficient strain had a reduced spore-forming ability.

### (4) Analysis of YMAF1 Protein Localization

To analyze the YMAF1 protein localization, first, western blotting was performed. Specifically, fungal cells (Afs35, d-YMAF1, or YMAF1-comp) cultured at 37°C in a minimal medium AMM were frozen with liquid nitrogen and disrupted. Then, the resultant was suspended in 50 mM Tris-HCl (pH 7.5), 100 mM NaCl, 10% glycerol, and a protease inhibitor (manufactured by Roche Applied Science). Subsequently, the suspension was centrifuged to prepare the obtained supernatant as a crude liquid cell extract, and the obtained precipitate as a fraction of the cell wall and the like (cell wall, cell membrane, and periplasm). Thereafter, these were subjected to SDS-PAGE, and then the YMAF1 protein was detected by western blotting using the anti-YMAF1 polyclonal antibody. Fig. 19 shows the obtained result.

As apparent from the result shown in Fig. 19, a YMAF1 protein having a molecular weight of approximately 60 kDa was detected from the fractions of the cell walls and the like of Afs35 and YMAF1-comp. Note that since the YMAF1 protein itself has a molecular weight of 23 kDa, it is inferred that the molecular weight was shifted by the modification of a carbohydrate or the like.

To analyze the YMAF1 protein localization, next, immunostaining was performed. Specifically, after Afs35 was fixed with 4% paraformaldehyde, the cells were caused to adhere to a glass plate coated with poly-L lysine. Then, the cells were fixed with methanol, followed by blocking treatment, and a treatment with the anti-YMAF1 polyclonal antibody. The YMAF1 protein was detected with Alexa Fluor 488 ANti-Rabbit SFX Kit (Alexa Fluor 594 GOAT ANti-Rabbit IgG SFX Kit, manufactured by Invitrogen Corp.). Fig. 20 shows the obtained result.

As apparent from the result shown in Fig. 20, by the immunostaining using the anti-YMAF1 polyclonal antibody, images were obtained, in which cell wall portions of the fungal cells with bud formation were stained.

Moreover, using a 24-well plate, conidia of *Aspergillus fumigatus* were added to media at 1.5 × 10⁴ conidia (the number of spores)/1 ml, followed by a treatment with the anti-YMAF1 polyclonal antibody at various concentrations. The resultant was cultured at 30°C for 14 hours, and then observed. Fig. 21 shows the obtained result.

As apparent from the result shown in Fig. 21, the wet weight of the fungal cells did not change in a manner dependent on the concentration of the anti-YMAF1 polyclonal antibody. Nevertheless, in the culture solution treated with the antibody, the fungal cell aggregation was no longer observed. The results suggested that: YMAF1 was mainly present on the cell wall surface as confirmed by the western blotting and the immunostaining; furthermore, YMAF1 was a protein contributing to the aggregation of fungal cells.

### (Example 5) Involvement of YMAF1 Protein in Pathogenicity of Mouse Model of Aspergillosis

To examine the association between the YMAF1 protein and aspergillosis in mice, 5 × 10⁶ spores of each of the parental strain Afs35, the YMAF1 gene-deficient strain, and the YMAF1 gene complementation strain were administered to bronchi of 7 individual ICR mice to examine the survival rate. Fig. 22 shows the obtained result.

As apparent from the result shown in Fig. 22, the mice to which Afs35 or the YMAF1 gene complementation strain was administered died within 4 to 10 days after the administration; meanwhile, in the case of the YMAF1 gene-deficient strain, no individual was observed to die after Day 4 unlike the other specimens. Thus, such a difference in the survival rate suggested that the YMAF1 protein was involved in the pathogenicity.

### (Example 6) Therapeutic (Life-Extending) Effect of Anti-YMAF1 Antibody

Using experimental mice having aspergillosis (invasive *Aspergillus* model mice), an therapeutic effect using an antibody was examined. Specifically, first, immuno suppression pretreatments were performed on ICR mice (8 weeks old, female) by subcutaneously administering 200 µg/kg of cortisone acetate on the day before the fungal inoculation, on the day of the inoculation, and on one day thereafter. Then, 50 µl of a spore suspension of an *A. Fummigatus* MF13 strain, 1 × 10⁸/ml, was administered into the trachea of the model mice. On the next day of the fungal inoculation, 150 µg of the anti-YMAF1 monoclonal antibody (4B6M2GK antibody) per individual was administered to the model mice to examine a change in the survival rate. Fig. 23 shows the obtained result.

As apparent from the result shown in Fig. 23, a life-extending effect was observed in the mice to which the 4B6M2GK antibody (4B6) was administered in comparison with a mouse IgG1 antibody (manufactured by ACTGen, Inc.) used as a control.

### (Example 7) Construction of YMAF1 sandwich ELISA System

The construction of a YMAF1 sandwich ELISA system was attempted, which could be suitably used in a diagnosis of aspergillosis by targeting the YMAF1 protein.

Specifically, first, the purified fraction of the anti-YMAF1 monoclonal antibody prepared in Example 3 (2) was mixed with a 10-fold molar amount of NHS-LC-biotin (manufactured by PIEACE), and reacted with each other for 4 hours under a light-shielded condition for biotinylation. After dialysis with PBS, a biotinylated monoclonal antibody was prepared. Meanwhile, 5 mg/ml of a protein GST-YMAF1-His6-Flag prepared with *Escherichia coli* was adsorbed to a 96-well micro plate at 50 µl/well and sensitized. Then, using the antigen plate thus prepared, the titer of the biotinylated anti-YMAF1 monoclonal antibody was checked to examine an appropriate concentration used as a secondary antibody in the sandwich ELISA system.

Next, to examine the sandwich ELISA conditions, an antibody sensitized plate was prepared using the unmodified anti-YMAF1 monoclonal antibody at certain concentrations as a primary antibody (capture antibody), and reacted with the recombinant protein (GST-YMAF1-His6-Flag) at concentrations of 1 µg/ml, 0.1 µg/ml, 0.01 µg/ml, and 0 µg/ml. Then, as a secondary reaction, a biotinylated anti-YMAF1 monoclonal antibody (secondary antibody, detection antibody) was reacted therewith at the appropriate concentration determined above. Further, as a tertiary reaction, Neutravidin-POD was reacted therewith. Then, a chromogenic enzyme substrate was added to develop a color, and the absorbance was measured.

In this manner, the construction of the sandwich ELISA system was attempted using all combinations of the four monoclonal antibodies prepared in Example 3 (2) as the primary antibody or the secondary antibody. However, no system was obtained, which demonstrated a dependency on the concentration of the YMAF1 recombinant protein.

For this reason, a rabbit was immunized with the YMAF1 recombinant protein to prepare a polyclonal antibody as described above.

Then, the construction of the sandwich ELISA system was attempted using the four anti-YMAF1 monoclonal antibodies as the primary antibody, and the anti-YMAF1 polyclonal antibody biotinylated by the same procedure as above as the secondary antibody. Figs. 24 and 25 show the result of evaluating the systems respectively using the 1B4C monoclonal antibody (1B4C) and the 3G4FB7 monoclonal antibody (3G4) as secondary antibody.

As apparent from the result shown in Figs. 24 and 25, even if any of the four monoclonal antibodies is used, although not all are shown in the figures, a dependency on the concentration of the YMAF1 recombinant protein was demonstrated. In addition, the sensitivity was 0.3 ng/ml or higher, and the systems with quite a high sensitivity were successfully constructed.

Next, *Aspergillus fumigatus* was shake-cultured at 30°C using various types of media. The amount of the YMAF1 protein in the culture supernatant was measured using the YMAF1 sandwich ELISA systems (the systems respectively using the 1B4C monoclonal antibody (1B4C) and the 3G4FB7 monoclonal antibody (3G4) as the primary antibody). Note that the composition of the Sabouraud medium used for culturing *Aspergillus fumigatus* included, per L, Pancreatic Digest of Casein 5.0 g, Peptic Digest of Animal Tissue 5.0 g, and dextrose 20.0 g. Figs. 26 and 27 show the obtained result.

As apparent from the result shown in Figs. 26 and 27, it was found out that the amount of the YMAF1 protein released into the culture supernatant varied depending on the type of the media.

### (Example 8) Antibody Variable Region-Determination Method

To clarify the gene sequences of variable regions of the 1B4C, 2G11GB5, 3G4FB7, 4B6M2GK monoclonal antibodies, 2 × 10⁶ of hybridoma cells producing the 1B4C, 2G11GB5, 3G4FB7, or 4B6M2GK antibodies were suspended in 1 ml of Trizol (manufactured by Invitrogen Corp., #15596-026) and left standing for 5 minutes, and 200 µl of chloroform was added thereto, followed by suspending for 15 seconds and then centrifugation at 12,000 × g for 15 minutes to obtain a supernatant. The supernatant was mixed with 500 µl of isopropanol, followed by centrifugation at 12,000 × g for 10 minutes. The resulting pellets were washed with 80% ethanol, and total RNA was obtained. Then, a total amount thereof was dissolved in 20 µl of water. A solution containing 5 µg of the total RNA was used. Using SuperScript(TM) Choice System, a double-stranded cDNA was prepared from the total RNA. The obtained double-stranded cDNA was subjected to an ethanol precipitation treatment. Then, using LigationHigh, the 5'-end and the 3'-end of the double-stranded cDNA were ligated, 1 µl of which was used as a template to perform PCR. Primers used were designed for constant regions of a heavy chain and a light chain. The primers had the following sequences.
<1B4C>
   Heavy chain 5' side GATACCCTGGATGACTTCAG (SEQ ID NO: 36 )
   Heavy chain 3' side CTCTCAGCATGGAAGGACAG (SEQ ID NO: 37 )
<2G11GB5 and 3G4FB7>
   Heavy chain 5' side AGGGTACAGTCACCAAGCTG (SEQ ID NO: 38 )
   Heavy chain 3' side TGCATGAGGCTCTCCATAAC (SEQ ID NO: 39 )
<4B6M2GK>
   Heavy chain 5' side TGGACAGGGATCCAGAGTTC (SEQ ID NO: 40 )
   Heavy chain 3' side CTGCTCTGTGTTACATGAGG (SEQ ID NO: 41 )
<Common>
   Light chain 5' side CACTGCCATCAATCTTCCAC (SEQ ID NO: 42 )
   Light chain 3' side TGTCAAGAGCTTCAACAGGA (SEQ ID NO: 43 ) .

The PCR products were each electrophoresed on a 1.5% gel, and then cut out for purification. Subsequently, using the purified DNAs, sequencing was performed. Moreover, as to the light chain, the sequencing was performed after the purified DNAs were cloned. As a result, it was found out that 2G11GB5 and 3G4FB7 had the same variable regions. Additionally, the base sequence of the light chain variable region of the 1B4C antibody thus determined is shown in SEQ ID NO: 23, and the amino acid sequence thereof is shown in SEQ ID NO: 24; the base sequence of the heavy chain variable region is shown in SEQ ID NO: 25, and the amino acid sequence thereof is shown in SEQ ID NO: 26 (see Figs. 28 and 29). Moreover, the base sequence of the light chain variable region of the 3G4FB7 antibody (2G11GB5 antibody) thus determined is shown in SEQ ID NO: 27, and the amino acid sequence thereof is shown in SEQ ID NO: 28; the base sequence of the heavy chain variable region is shown in SEQ ID NO: 29, and the amino acid sequence thereof is shown in SEQ ID NO: 30 (see Figs. 30 and 31). Further, the base sequence of the light chain variable region of the 4B6M2GK antibody thus determined is shown in SEQ ID NO: 19, and the amino acid sequence thereof is shown in SEQ ID NO: 20; the base sequence of the heavy chain variable region is shown in SEQ ID NO: 21, and the amino acid sequence thereof is shown in SEQ ID NO: 22 (see Figs. 32 and 33).

In addition, the amino acid sequences of these variable regions were numbered utilizing the sequence analysis in the site "Andrew C.R. Martin's Bioinformatics Group" of UCL (http://www.bioinf.org.uk/abysis/tools/analyze.cgi). CDR regions were identified according to the standard described in "Table of CDR Definitions" (http://www.bioinf.org.uk/abs/#kabatnum). Figs. 29, 31, and 33 show the result of CDR prediction and signal sequences of the light and heavy chains. Moreover, the amino acid sequences of the light chain CDR1, CDR2, and CDR3 of the 1B4C antibody are shown in SEQ ID NOs: 7 to 9, and the amino acid sequences of the heavy chain CDR1, CDR2, and CDR3 are shown in SEQ ID NO: 10 to 12. Further, the amino acid sequences of the light chain CDR1, CDR2, and CDR3 of the 3G4FB7 antibody (2G11GB5 antibody) are shown in SEQ ID NOs: 13 to 15, and the amino acid sequences of the heavy chain CDR1, CDR2, and CDR3 are shown in SEQ ID NOs : 16 to 18. Furthermore, the amino acid sequences of the light chain CDR1, CDR2, and CDR3 of the 4B6M2GK antibody are shown in SEQ ID NOs: 1 to 3, and the amino acid sequences of the heavy chain CDR1, CDR2, and CDR3 are shown in SEQ ID NOs: 4 to 6.

### (Example 9) Epitope Analysis of Monoclonal Antibodies

To specify epitopes of the 1B4C, 3G4FB7 (2G11GB5), 4B6M2GK monoclonal antibodies, Ba/F3 cells expressing various YMAF1 polypeptides of different chain lengths were prepared, and the reactivities with the antibodies were evaluated.

Specifically, polypeptides respectively consisting of 33aa (indicating the chain length from the N-terminus. The same shall apply hereinafter), 63aa, 93aa, 123aa, 153aa, and 183aa of YMAF1 were targeted for the analysis. Then, using a recombinant plasmid containing full-length YMAF1 as a template, using DNAs having the following sequences as primers, and using PrimeSTAR MAX DNA polymerase (manufactured by Takara Bio Inc., #R045A) as a polymerase, genes encoding the seven polypeptides were isolated.

Forward primer (SEQ ID NO: 44): GCACTCCGTTCTGGATAATG Reverse primers (the number added to Rmeans the chain length of a polypeptide encoded by an amplification product) R33 (SEQ ID NO: 45):
TTTTCCTTTTGCGGCCGCCCCGGCGGGCGCTGTTGTCTGCGCAGGAGG R63 (SEQ ID NO: 46):
TTTTCCTTTTGCGGCCGCTGTGGTCGTGGGGCTGGGCTCCTCGTCACG R93 (SEQ ID NO: 47):
TTTTCCTTTTGCGGCCGCGTAGTGACCATAGTCCCCATATTGACCATA R123 (SEQ ID NO: 48):
TTTTCCTTTTGCGGCCGCATATTGACCATAGTTTCCGTAGTTTGCTGG R153 (SEQ ID NO: 49):
TTTTCCTTTTGCGGCCGCGCCGTAGTCGGCGGGAGTGGGAGAGGGAGT R183 (SEQ ID NO: 50):
TTTTCCTTTTGCGGCCGCGGTGGTAGTCGTGCGAGGCTCGTCGTCTCT.

The obtained PCR products were each electrophoresed on a 1% agarose gel, and then cut out for purification, followed by a restriction enzyme treatment with EcoRI and NotI. Moreover, pMX-SST was also subjected to the restriction enzyme treatment with EcoRI and NotI, and cut out for purification. Further, both were treated with LigationHigh, and plasmids having the PCR products inserted were prepared. Then, the plasmids were each introduced into *Escherichia coli,* which was plated on an LB agarose plate containing 50 µg of ampicillin. Subsequently, colonies were obtained by culturing at 37°C overnight, and PCR was performed thereon in such a manner that the inserted portion of the plasmid was amplified. Whether the plasmid was a pMX-SST vector containing a desired sequence was checked by sequencing. As PCR primer for the sequencing, the following oligonucleotides were used.
SST3' side 5'-GGCGCGCAGCTGTAAACGGTAG-3' (SEQ ID NO: 51)
SST5' side 5'-CGGGGGTGGACCATCCTCTA-3' (SEQ ID NO: 52).

After that, by the same method as in the virus packaging and thereafter described in Example 1 (4), Ba/F3 cells containing DNA sequences of the YMAF1 genes of various chain lengths (SST clones: ACT251-1 to ACT251-6) were prepared. Then, by the same procedure as the method described in Example 3 (2), the reactivities between ACT251-1 to ACT251-6 and the 1B4C, 3G4FB7 (2G11GB5), and 4B6M2GK antibodies were analyzed with a flow cytometer. Figs. 34 to 36 show the obtained result.

As apparent from the result shown in Figs. 34 to 36, all of the 1B4C, 3G4FB7 (2G11GB5), and 4B6M2GK antibodies showed the reactivities with the clone expressing the polypeptide consisting of 1 to 33 amino acids from the N-terminal side of the YMAF1 protein. Thus, it was revealed that the epitopes of these antibodies were contained between positions 1 and 33 from the N-terminus of the YMAF1 protein.

### [Industrial Applicability]

As described above, the present invention makes it possible to provide a method for testing an *Aspergillus fumigatus* infection, the method being capable of detecting *Aspergillus fumigatus* with a high sensitivity, and a composition for the testing. Moreover, it becomes possible to provide methods for preventing and treating an *Aspergillus fumigatus* infection, and a composition for the prevention and treatment. Furthermore, it becomes possible to provide a screening method for a compound useful in these methods, and an antibody useful in these methods.

Thus, the present invention is useful in testing, preventing, and treating chronic necrotizing pulmonary aspergillosis (CNPA) and the like.

### [Sequence Listing Free Text]

SEQ ID NO: 1
   <223> 4B6M2K antibody light chain variable region CDR1
SEQ ID NO: 2
   <223> 4B6M2K antibody light chain variable region CDR2
SEQ ID NO: 3
   <223> 4B6M2K antibody light chain variable region CDR3
SEQ ID NO: 4
   <223> 4B6M2K antibody heavy chain variable region CDR1
SEQ ID NO: 5
   <223> 4B6M2K antibody heavy chain variable region CDR2
SEQ ID NO: 6
   <223> 4B6M2K antibody heavy chain variable region CDR3
SEQ ID NO: 7
   <223> 1B4C antibody light chain variable region CDR1
SEQ ID NO: 8
   <223> 1B4C antibody light chain variable region CDR2
SEQ ID NO: 9
   <223> 1B4C antibody light chain variable region CDR3
SEQ ID NO: 10
   <223> 1B4C antibody heavy chain variable region CDR1
SEQ ID NO: 11
   <223> 1B4C antibody heavy chain variable region CDR2
SEQ ID NO: 12
   <223> 1B4C antibody heavy chain variable region CDR3
SEQ ID NO: 13
   <223> 3G4FB7 antibody light chain variable region CDR1
SEQ ID NO: 14
   <223> 3G4FB7 antibody light chain variable region CDR2
SEQ ID NO: 15
   <223> 3G4FB7 antibody light chain variable region CDR3
SEQ ID NO: 16
   <223> 3G4FB7 antibody heavy chain variable region CDR1
SEQ ID NO: 17
   <223> 3G4FB7 antibody heavy chain variable region CDR2
SEQ ID NO: 18
   <223> 3G4FB7 antibody heavy chain variable region CDR3
SEQ ID NO: 19
   <223> 4B6M2K antibody light chain variable region cDNA
SEQ ID NO: 21
   <223> 4B6M2K antibody heavy chain variable region cDNA
SEQ ID NO: 23
   <223> 1B4C antibody light chain variable region cDNA
SEQ ID NO: 25
   <223> 1B4C antibody heavy chain variable region cDNA
SEQ ID NO: 27
   <223> 3G4FB7 antibody light chain variable region cDNA
SEQ ID NO: 29
   <223> 3G4FB7 antibody heavy chain variable region cDNA
SEQ ID NOs: 34 to 52
   <223> artificially synthesized primer sequences

## Claims

1. A method for testing an *Aspergillus fumigatus* infection, comprising a step of detecting a presence of a YMAF1 protein in a biological sample separated from a subject.

2. The method according to claim 1, wherein the presence of the YMAF1 protein is detected using an antibody against the YMAF1 protein.

3. A composition for testing an *Aspergillus fumigatus* infection, comprising an antibody against a YMAF1 protein.

4. A method for preventing or treating an *Aspergillus fumigatus* infection, comprising a step of administering an antibody against a YMAF1 protein.

5. A composition for preventing or treating an *Aspergillus fumigatus* infection, comprising an antibody against a YMAF1 protein.

6. A screening method for a compound for testing, preventing, or treating an *Aspergillus fumigatus* infection, the method comprising the steps of:
bringing a test compound into contact with any one of a YMAF1 protein and a portion thereof; and
selecting a compound bound to any one of the YMAF1 protein and the portion thereof.

7. An antibody capable of recognizing a region comprising the amino acid sequence of SEQ ID NO: 33 in a YMAF1 protein.

8. An antibody according to any one of the following (a) to (c):
(a) an antibody capable of binding to a YMAF1 protein, and comprising
a light chain variable region including amino acid sequences of SEQ ID NOs: 1 to 3 or the amino acid sequences in at least any one of which one or more amino acids are substituted, deleted, added, and/or inserted, and
a heavy chain variable region including amino acid sequences of SEQ ID NOs: 4 to 6 or the amino acid sequences in at least any one of which one or more amino acids are substituted, deleted, added, and/or inserted;
(b) an antibody capable of binding to the YMAF1 protein, and comprising
a light chain variable region including amino acid sequences of SEQ ID NOs: 7 to 9 or the amino acid sequences in at least any one of which one or more amino acids are substituted, deleted, added, and/or inserted, and
a heavy chain variable region including amino acid sequences of SEQ ID NOs: 10 to 12 or the amino acid sequences in at least any one of which one or more amino acids are substituted, deleted, added, and/or inserted; and
(c) an antibody capable of binding to the YMAF1 protein, and comprising
a light chain variable region including amino acid sequences of SEQ ID NOs: 13 to 15 or the amino acid sequences in at least any one of which one or more amino acids are substituted, deleted, added, and/or inserted, and
a heavy chain variable region including amino acid sequences of SEQ ID NOs: 16 to 18 or the amino acid sequences in at least any one of which one or more amino acids are substituted, deleted, added, and/or inserted.

9. An antibody according to any one of the following (a) to (c):
(a) an antibody capable of binding to a YMAF1 protein, and comprising
a light chain variable region including the amino acid sequence of SEQ ID NO: 20, the amino acid sequence from which a signal sequence is removed, or at least any one of these amino acid sequences in which one or more amino acids are substituted, deleted, added, and/or inserted, and
a heavy chain variable region including the amino acid sequence of SEQ ID NO: 22, the amino acid sequence from which a signal sequence is removed, or at least any one of these amino acid sequences in which one or more amino acids are substituted, deleted, added, and/or inserted;
(b) an antibody capable of binding to the YMAF1 protein, and comprising
a light chain variable region including the amino acid sequence of SEQ ID NO: 24, the amino acid sequence from which a signal sequence is removed, or at least any one of these amino acid sequences in which one or more amino acids are substituted, deleted, added, and/or inserted, and
a heavy chain variable region including the amino acid sequence of SEQ ID NO: 26, the amino acid sequence from which a signal sequence is removed, or at least any one of these amino acid sequences in which one or more amino acids are substituted, deleted, added, and/or inserted; and
(c) an antibody capable of binding to the YMAF1 protein, and comprising
a light chain variable region including the amino acid sequence of SEQ ID NO: 28, the amino acid sequence from which a signal sequence is removed, or at least any one of these amino acid sequences in which one or more amino acids are substituted, deleted, added, and/or inserted, and
a heavy chain variable region including the amino acid sequence of SEQ ID NO: 30, the amino acid sequence from which a signal sequence is removed, or at least any one of these amino acid sequences in which one or more amino acids are substituted, deleted, added, and/or inserted.


SEQUENCE LISTING

<110>  National Institute of Infections Disease
       ACTGen, Inc.
<120>  METHODS AND COMPOSITIONS FOR EXAMINATION, PREVENTION, AND TREATMENT
OF ASPERGILLUS FUMIGATUS INFECTION
<130>  APC004P1
<160>  56
<170>  PatentIn version 3.1

<210>  1
<211>  17
<212>  PRT
<213>  Mus musculus
<220>
<221>  SITE
<222>  (1)..(17)
<223>  4B6M2K Lv CDR1
<400>  1
Lys Ser Ser Gln Ser Leu Leu Asn Ser Asn Asn Gln Lys Asn Tyr Leu
1               5                   10                  15
Ala

<210>  2
<211>  7
<212>  PRT
<213>  Mus musculus
<220>
<221>  SITE
<222>  (1)..(7)
<223>  4B6M2K Lv CDR2
<400>  2
Phe Ala Ser Thr Arg Asp Ser
1               5

<210>  3
<211>  9
<212>  PRT
<213>  Mus musculus
<220>
<221>  SITE
<222>  (1)..(9)
<223>  4B6M2K Lv CDR3
<400>  3
Gln Gln His Tyr Ser Thr Pro Tyr Thr
1               5

<210>  4
<211>  5
<212>  PRT
<213>  Mus musculus
<220>
<221>  SITE
<222>  (1)..(5)
<223>  4B6M2K Hv CDR1
<400>  4
Ser Tyr Ala Met Ser
1               5

<210>  5
<211>  17
<212>  PRT
<213>  Mus musculus

<220>
<221>  SITE
<222>  (1)..(17)
<223>  4B6M2K Hv CDR2
<400>  5
Thr Ile Ser Ser Gly Gly Thr Tyr Thr Tyr Tyr Pro Asp Ser Val Lys
1               5                   10                  15
Gly

<210>  6
<211>  6
<212>  PRT
<213>  Mus musculus
<220>
<221>  SITE
<222>  (1)..(6)
<223>  4B6M2K Hv CDR3
<400>  6
Gly Met Pro Gly Asp Tyr
1               5

<210>  7
<211>  11
<212>  PRT
<213>  Mus musculus
<220>
<221>  SITE
<222>  (1)..(11)
<223>  1B4C Lv CDR1
<400>  7
Arg Ala Ser Gln Glu Ile Ser Gly Tyr Leu Ser
1               5                   10

<210>  8
<211>  7
<212>  PRT
<213>  Mus musculus
<220>
<221>  SITE
<222>  (1)..(7)
<223>  1B4C Lv CDR2
<400>  8
Ala Ala Ser Thr Leu Asp Ser
1               5

<210>  9
<211>  9
<212>  PRT
<213>  Mus musculus
<220>
<221>  SITE
<222>  (1)..(9)
<223>  1B4C Lv CDR3
<400>  9
Leu Gln Tyr Ala Ser Tyr Pro Leu Thr
1               5

<210>  10
<211>  5
<212>  PRT
<213>  Mus musculus
<220>
<221>  SITE

<222>  (1)..(5)
<223>  1B4C Hv CDR1
<400>  10
Ser Ser Trp Met Asn
1               5

<210>  11
<211>  17
<212>  PRT
<213>  Mus musculus
<220>
<221>  SITE
<222>  (1)..(17)
<223>  1B4C Hv CDR2
<400>  11
Arg Ile Tyr Pro Gly Asp Gly Asp Thr Asn Tyr Asn Gly Lys Phe Lys
1               5                   10                  15
Gly

<210>  12
<211>  13
<212>  PRT
<213>  Mus musculus
<220>
<221>  SITE
<222>  (1)..(13)
<223>  1B4C Hv CDR3
<400>  12
Met Arg Gly Leu Arg Arg Asp Tyr Tyr Ala Met Asp Tyr
1               5                   10

<210>  13
<211>  15
<212>  PRT
<213>  Mus musculus
<220>
<221>  SITE
<222>  (1)..(15)
<223>  3G4FB7 Lv CDR1
<400>  13
Arg Ala Ser Glu Ser Val Ala Ser Tyr Gly Asn Ser Phe Met His
1               5                   10                  15

<210>  14
<211>  7
<212>  PRT
<213>  Mus musculus
<220>
<221>  SITE
<222>  (1)..(7)
<223>  3G4FB7 Lv CDR2
<400>  14
Leu Ala Ser Asn Leu Glu Ser
1               5

<210>  15
<211>  9
<212>  PRT
<213>  Mus musculus
<220>
<221>  SITE
<222>  (1)..(9)
<223>  3G4FB7 Lv CDR3

<400>  15
Gln Gln Asn Asn Glu Asp Pro Tyr Thr
1               5

<210>  16
<211>  5
<212>  PRT
<213>  Mus musculus
<220>
<221>  SITE
<222>  (1)..(5)
<223>  3G4FB7 Hv CDR1
<400>  16
Ala Tyr Trp Ile His
1               5

<210>  17
<211>  17
<212>  PRT
<213>  Mus musculus
<220>
<221>  SITE
<222>  (1)..(17)
<223>  3G4FB7 Hv CDR2
<400>  17
Tyr Ile Asn Pro Thr Thr Asp Tyr Thr Glu Tyr Asn Gln Arg Phe Lys
1               5                   10                  15
Asp

<210>  18
<211>  13
<212>  PRT
<213>  Mus musculus
<220>
<221>  SITE
<222>  (1)..(13)
<223>  3G4FB7 Hv CDR3
<400>  18
Glu Glu Asp Tyr Asp Gly Gly His Tyr Ala Met Asp Tyr
1               5                   10

<210>  19
<211>  405
<212>  DNA
<213>  Mus musculus
<220>
<221>  CDS
<222>  (1)..(405)
<223>  4B6M2K Lv cDNA
<400>  19
atg gaa tca cag acc cag gtc ctc atg ttt ctt ctg ctc tgg gta tct       48
Met Glu Ser Gln Thr Gln Val Leu Met Phe Leu Leu Leu Trp Val Ser
1               5                   10                  15
ggt gcc tgt gca gac att ttg atg act cag tct cca tcc tcc ctg gct       96
Gly Ala Cys Ala Asp Ile Leu Met Thr Gln Ser Pro Ser Ser Leu Ala
            20                  25                  30
atg tca gtg gga cag aag gtc act atg agc tgc aag tcc agt cag agc      144
Met Ser Val Gly Gln Lys Val Thr Met Ser Cys Lys Ser Ser Gln Ser
        35                  40                  45
ctt tta aat agt aac aat caa aag aac tat ttg gcc tgg tac cag cag      192
Leu Leu Asn Ser Asn Asn Gln Lys Asn Tyr Leu Ala Trp Tyr Gln Gln
    50                  55                  60
aaa cca gga cag tct cct aaa ctt ctg gta ttc ttt gca tcc act agg      240

Lys Pro Gly Gln Ser Pro Lys Leu Leu Val Phe Phe Ala Ser Thr Arg
65                  70                  75                  80
gat tct ggg gtc cct gat cgc ttc ata ggc agt ggc tct ggg aca gat       288
Asp Ser Gly Val Pro Asp Arg Phe Ile Gly Ser Gly Ser Gly Thr Asp
                85                  90                  95
ttc act ctt acc atc agc act gtg cag gct gag gac ctg gca gat tac       336
Phe Thr Leu Thr Ile Ser Thr Val Gln Ala Glu Asp Leu Ala Asp Tyr
            100                 105                 110
ttc tgt cag caa cat tat agc act ccg tac acg ttc gga ggg ggg acc       384
Phe Cys Gln Gln His Tyr Ser Thr Pro Tyr Thr Phe Gly Gly Gly Thr
        115                 120                 125
aag ctg gaa ata aaa cgg gct                                           405
Lys Leu Glu Ile Lys Arg Ala
    130                 135

<210>  20
<211>  135
<212>  PRT
<213>  Mus musculus
<400>  20
Met Glu Ser Gln Thr Gln Val Leu Met Phe Leu Leu Leu Trp Val Ser
1               5                   10                  15
Gly Ala Cys Ala Asp Ile Leu Met Thr Gln Ser Pro Ser Ser Leu Ala
            20                  25                  30
Met Ser Val Gly Gln Lys Val Thr Met Ser Cys Lys Ser Ser Gln Ser
        35                  40                  45
Leu Leu Asn Ser Asn Asn Gln Lys Asn Tyr Leu Ala Trp Tyr Gln Gln
    50                  55                  60
Lys Pro Gly Gln Ser Pro Lys Leu Leu Val Phe Phe Ala Ser Thr Arg
65                  70                  75                  80
Asp Ser Gly Val Pro Asp Arg Phe Ile Gly Ser Gly Ser Gly Thr Asp
                85                  90                  95
Phe Thr Leu Thr Ile Ser Thr Val Gln Ala Glu Asp Leu Ala Asp Tyr
            100                 105                 110
Phe Cys Gln Gln His Tyr Ser Thr Pro Tyr Thr Phe Gly Gly Gly Thr
        115                 120                 125
Lys Leu Glu Ile Lys Arg Ala
    130                 135

<210>  21
<211>  402
<212>  DNA
<213>  Mus musculus
<220>
<221>  CDS
<222>  (1)..(402)
<223>  4B6M2K Hv cDNA
<400>  21
atg aac ttc ggg ctc agc ttg att ttc ctt gtc ctt gtt tta aaa ggt        48
Met Asn Phe Gly Leu Ser Leu Ile Phe Leu Val Leu Val Leu Lys Gly
1               5                   10                  15
gtc cag tgt gaa gtg atg ctg gtg gag tct ggg gga ggc tta gtg aag        96
Val Gln Cys Glu Val Met Leu Val Glu Ser Gly Gly Gly Leu Val Lys
            20                  25                  30
cct gga ggg tcc ctg aaa ctc tcc tgt gca gcc tct gga ttc act ttc       144
Pro Gly Gly Ser Leu Lys Leu Ser Cys Ala Ala Ser Gly Phe Thr Phe
        35                  40                  45
agt agc tat gcc atg tct tgg gtt cgc cag act ccg gag aag agg ctg       192
Ser Ser Tyr Ala Met Ser Trp Val Arg Gln Thr Pro Glu Lys Arg Leu
    50                  55                  60
gag tgg gtc gca acc att agt agt ggt ggt act tac acc tac tat cca       240
Glu Trp Val Ala Thr Ile Ser Ser Gly Gly Thr Tyr Thr Tyr Tyr Pro
65                  70                  75                  80

gac agt gtg aag ggg cga ttc acc atc tcc aga gac aat gcc aag aac      288
Asp Ser Val Lys Gly Arg Phe Thr Ile Ser Arg Asp Asn Ala Lys Asn
                85                  90                  95
acc ctg tac ctg caa atg agc agt ctg agg tct gag gac acg gcc atg      336
Thr Leu Tyr Leu Gln Met Ser Ser Leu Arg Ser Glu Asp Thr Ala Met
            100                 105                 110
tat tac tgt gga aga ggg atg ccg ggt gac tac tgg ggc caa ggc acc      384
Tyr Tyr Cys Gly Arg Gly Met Pro Gly Asp Tyr Trp Gly Gln Gly Thr
        115                 120                 125
act ctc aca gtc tcc tca                                              402
Thr Leu Thr Val Ser Ser
    130

<210>  22
<211>  134
<212>  PRT
<213>  Mus musculus
<400>  22
Met Asn Phe Gly Leu Ser Leu Ile Phe Leu Val Leu Val Leu Lys Gly
1               5                   10                  15
Val Gln Cys Glu Val Met Leu Val Glu Ser Gly Gly Gly Leu Val Lys
            20                  25                  30
Pro Gly Gly Ser Leu Lys Leu Ser Cys Ala Ala Ser Gly Phe Thr Phe
        35                  40                  45
Ser Ser Tyr Ala Met Ser Trp Val Arg Gln Thr Pro Glu Lys Arg Leu
    50                  55                  60
Glu Trp Val Ala Thr Ile Ser Ser Gly Gly Thr Tyr Thr Tyr Tyr Pro
65                  70                  75                  80
Asp Ser Val Lys Gly Arg Phe Thr Ile Ser Arg Asp Asn Ala Lys Asn
                85                  90                  95
Thr Leu Tyr Leu Gln Met Ser Ser Leu Arg Ser Glu Asp Thr Ala Met
            100                 105                 110
Tyr Tyr Cys Gly Arg Gly Met Pro Gly Asp Tyr Trp Gly Gln Gly Thr
        115                 120                 125
Thr Leu Thr Val Ser Ser
    130

<210>  23
<211>  393
<212>  DNA
<213>  Mus musculus
<220>
<221>  CDS
<222>  (1)..(393)
<223>  1B4C Lv cDNA
<400>  23
atg gac atg agg gtt cct gct cac gtt ttt ggc ttc ttg ttg ctc tgg      48
Met Asp Met Arg Val Pro Ala His Val Phe Gly Phe Leu Leu Leu Trp
1               5                   10                  15
ttt cca ggt acc aga tgt gac atc cag atg acc cag tct cca tcc tcc      96
Phe Pro Gly Thr Arg Cys Asp Ile Gln Met Thr Gln Ser Pro Ser Ser
            20                  25                  30
tta tct gcc tct ctg gga gaa aga gtc agt ctc act tgt cgg gca agt      144
Leu Ser Ala Ser Leu Gly Glu Arg Val Ser Leu Thr Cys Arg Ala Ser
        35                  40                  45
cag gaa att agt ggt tac tta agc tgg ctt cag cag aaa cca gat gga      192
Gln Glu Ile Ser Gly Tyr Leu Ser Trp Leu Gln Gln Lys Pro Asp Gly
    50                  55                  60
act att aaa cgc ctg atc tac gcc gca tcc act tta gat tct ggt gtc      240
Thr Ile Lys Arg Leu Ile Tyr Ala Ala Ser Thr Leu Asp Ser Gly Val
65                  70                  75                  80
cca aaa agg ttc agt ggc agt agg tct ggg tca gat tat tct ctc acc      288
Pro Lys Arg Phe Ser Gly Ser Arg Ser Gly Ser Asp Tyr Ser Leu Thr

                85                  90                  95
atc agc agc ctt gag tct gaa gat ttt gca gac tat tac tgt cta caa      336
Ile Ser Ser Leu Glu Ser Glu Asp Phe Ala Asp Tyr Tyr Cys Leu Gln
            100                 105                 110
tat gct agt tat cct ctc acg ttc ggt gct ggg acc aag ctg gag ctg      384
Tyr Ala Ser Tyr Pro Leu Thr Phe Gly Ala Gly Thr Lys Leu Glu Leu
        115                 120                 125
aaa cgg gct                                                          393
Lys Arg Ala
    130

<210>  24
<211>  131
<212>  PRT
<213>  Mus musculus
<400>  24
Met Asp Met Arg Val Pro Ala His Val Phe Gly Phe Leu Leu Leu Trp
1               5                   10                  15
Phe Pro Gly Thr Arg Cys Asp Ile Gln Met Thr Gln Ser Pro Ser Ser
            20                  25                  30
Leu Ser Ala Ser Leu Gly Glu Arg Val Ser Leu Thr Cys Arg Ala Ser
        35                  40                  45
Gln Glu Ile Ser Gly Tyr Leu Ser Trp Leu Gln Gln Lys Pro Asp Gly
    50                  55                  60
Thr Ile Lys Arg Leu Ile Tyr Ala Ala Ser Thr Leu Asp Ser Gly Val
65                  70                  75                  80
Pro Lys Arg Phe Ser Gly Ser Arg Ser Gly Ser Asp Tyr Ser Leu Thr
                85                  90                  95
Ile Ser Ser Leu Glu Ser Glu Asp Phe Ala Asp Tyr Tyr Cys Leu Gln
            100                 105                 110
Tyr Ala Ser Tyr Pro Leu Thr Phe Gly Ala Gly Thr Lys Leu Glu Leu
        115                 120                 125
Lys Arg Ala
    130

<210>  25
<211>  423
<212>  DNA
<213>  Mus musculus
<220>
<221>  CDS
<222>  (1)..(423)
<223>  1B4C Hv cDNA
<400>  25
atg gaa tgg cct ttg atc ttt ctc ttc ctc ctg tca gga act gca ggt       48
Met Glu Trp Pro Leu Ile Phe Leu Phe Leu Leu Ser Gly Thr Ala Gly
1               5                   10                  15
gtc cac tcc cag gtt cag ctg cag cag tct gga cct gag ctg gtg aag       96
Val His Ser Gln Val Gln Leu Gln Gln Ser Gly Pro Glu Leu Val Lys
            20                  25                  30
cct ggg gcc tca gtg aag att tcc tgc aag gct tct ggc tat gca ttc      144
Pro Gly Ala Ser Val Lys Ile Ser Cys Lys Ala Ser Gly Tyr Ala Phe
        35                  40                  45
agt agc tcc tgg atg aac tgg gtg aag cag agg cct gga aag ggt ctt      192
Ser Ser Ser Trp Met Asn Trp Val Lys Gln Arg Pro Gly Lys Gly Leu
    50                  55                  60
gag tgg att gga cgg att tat cct gga gat gga gat act aac tac aat      240
Glu Trp Ile Gly Arg Ile Tyr Pro Gly Asp Gly Asp Thr Asn Tyr Asn
65                  70                  75                  80
ggg aag ttc aag ggc aag gcc aca ctg act gca gac aaa tcc tcc agc      288
Gly Lys Phe Lys Gly Lys Ala Thr Leu Thr Ala Asp Lys Ser Ser Ser
                85                  90                  95
aca gcc tac atg caa ctc agc agc ctg aca tct gag gac tct gcg gtc      336

Thr Ala Tyr Met Gln Leu Ser Ser Leu Thr Ser Glu Asp Ser Ala Val
            100                 105                 110
tac ttc tgt gca act atg cgg gga tta cga cgg gat tac tat gct atg       384
Tyr Phe Cys Ala Thr Met Arg Gly Leu Arg Arg Asp Tyr Tyr Ala Met
        115                 120                 125
gac tac tgg ggt caa gga acc tca gtc acc gtc tcc tca                   423
Asp Tyr Trp Gly Gln Gly Thr Ser Val Thr Val Ser Ser
    130                 135                 140

<210>  26
<211>  141
<212>  PRT
<213>  Mus musculus
<400>  26
Met Glu Trp Pro Leu Ile Phe Leu Phe Leu Leu Ser Gly Thr Ala Gly
1               5                   10                  15
Val His Ser Gln Val Gln Leu Gln Gln Ser Gly Pro Glu Leu Val Lys
            20                  25                  30
Pro Gly Ala Ser Val Lys Ile Ser Cys Lys Ala Ser Gly Tyr Ala Phe
        35                  40                  45
Ser Ser Ser Trp Met Asn Trp Val Lys Gln Arg Pro Gly Lys Gly Leu
    50                  55                  60
Glu Trp Ile Gly Arg Ile Tyr Pro Gly Asp Gly Asp Thr Asn Tyr Asn
65                  70                  75                  80
Gly Lys Phe Lys Gly Lys Ala Thr Leu Thr Ala Asp Lys Ser Ser Ser
                85                  90                  95
Thr Ala Tyr Met Gln Leu Ser Ser Leu Thr Ser Glu Asp Ser Ala Val
            100                 105                 110
Tyr Phe Cys Ala Thr Met Arg Gly Leu Arg Arg Asp Tyr Tyr Ala Met
        115                 120                 125
Asp Tyr Trp Gly Gln Gly Thr Ser Val Thr Val Ser Ser
    130                 135                 140

<210>  27
<211>  399
<212>  DNA
<213>  Mus musculus
<220>
<221>  CDS
<222>  (1)..(399)
<223>  3G4FB7 Lv cDNA
<400>  27
atg gag aca gac aca ctc ctg cta tgg gtg ctg ctg ctc tgg gtt cca        48
Met Glu Thr Asp Thr Leu Leu Leu Trp Val Leu Leu Leu Trp Val Pro
1               5                   10                  15
ggt tcc aca ggt aac att gtg ctg acc caa tct cca gct tct ttg gct        96
Gly Ser Thr Gly Asn Ile Val Leu Thr Gln Ser Pro Ala Ser Leu Ala
            20                  25                  30
gtg tct cta ggg cag agg gcc acc ata tcc tgc aga gcc agt gaa agt       144
Val Ser Leu Gly Gln Arg Ala Thr Ile Ser Cys Arg Ala Ser Glu Ser
        35                  40                  45
gtt gct agt tat ggc aat agt ttt atg cac tgg tac cag cag aaa cca       192
Val Ala Ser Tyr Gly Asn Ser Phe Met His Trp Tyr Gln Gln Lys Pro
    50                  55                  60
gga cag cca ccc aaa ctc ctc atc tat ctt gca tcc aac cta gaa tct       240
Gly Gln Pro Pro Lys Leu Leu Ile Tyr Leu Ala Ser Asn Leu Glu Ser
65                  70                  75                  80
ggg gtc cct gcc agg ttc agt ggc agt ggg tct agg aca gac ttc acc       288
Gly Val Pro Ala Arg Phe Ser Gly Ser Gly Ser Arg Thr Asp Phe Thr
                85                  90                  95
ctc acc att gat cct gtg gag gct gat gat gct gca acc tat tac tgt       336
Leu Thr Ile Asp Pro Val Glu Ala Asp Asp Ala Ala Thr Tyr Tyr Cys
            100                 105                 110

cag caa aat aat gag gat ccg tac acg ttc gga ggg ggg acc aag gtg 384
Gln Gln Asn Asn Glu Asp Pro Tyr Thr Phe Gly Gly Gly Thr Lys Val
        115                 120                 125
gaa ata aaa cgg gct 399
Glu Ile Lys Arg Ala
    130

<210> 28
<211> 133
<212> PRT
<213> Mus musculus
<400> 28
Met Glu Thr Asp Thr Leu Leu Leu Trp Val Leu Leu Leu Trp Val Pro
1               5                   10                  15
Gly Ser Thr Gly Asn Ile Val Leu Thr Gln Ser Pro Ala Ser Leu Ala
            20                  25                  30
Val Ser Leu Gly Gln Arg Ala Thr Ile Ser Cys Arg Ala Ser Glu Ser
        35                  40                  45
Val Ala Ser Tyr Gly Asn Ser Phe Met His Trp Tyr Gln Gln Lys Pro
    50                  55                  60
Gly Gln Pro Pro Lys Leu Leu Ile Tyr Leu Ala Ser Asn Leu Glu Ser
65                  70                  75                  80
Gly Val Pro Ala Arg Phe Ser Gly Ser Gly Ser Arg Thr Asp Phe Thr
                85                  90                  95
Leu Thr Ile Asp Pro Val Glu Ala Asp Asp Ala Ala Thr Tyr Tyr Cys
            100                 105                 110
Gln Gln Asn Asn Glu Asp Pro Tyr Thr Phe Gly Gly Gly Thr Lys Val
        115                 120                 125
Glu Ile Lys Arg Ala
    130

<210> 29
<211> 423
<212> DNA
<213> Mus musculus
<220>
<221> CDS
<222> (1)..(423)
<223> 3G4FB7 Hv cDNA
<400> 29
atg gaa agg cac tgg atc ttt ctc ttc ctg ttt tca gta cct gca ggt 48
Met Glu Arg His Trp Ile Phe Leu Phe Leu Phe Ser Val Pro Ala Gly
1               5                   10                  15
gtc cgc tcc cag gtc cag ctt cag cag tct ggg gct gaa ctg gca aaa 96
Val Arg Ser Gln Val Gln Leu Gln Gln Ser Gly Ala Glu Leu Ala Lys
            20                  25                  30
cct ggg gcc tca gtg aag atg tcc tgc aag act tct ggc tac acc ttt 144
Pro Gly Ala Ser Val Lys Met Ser Cys Lys Thr Ser Gly Tyr Thr Phe
        35                  40                  45
act gcc tac tgg ata cac tgg gta aaa cag agg cct gga cag ggt ctg 192
Thr Ala Tyr Trp Ile His Trp Val Lys Gln Arg Pro Gly Gln Gly Leu
    50                  55                  60
gag tgg att gga tac att aat cct acc act gat tat act gag tac aat 240
Glu Trp Ile Gly Tyr Ile Asn Pro Thr Thr Asp Tyr Thr Glu Tyr Asn
65                  70                  75                  80
cag aga ttc aag gac aag gcc aca ttg act gca gac aaa tcc tcc aac 288
Gln Arg Phe Lys Asp Lys Ala Thr Leu Thr Ala Asp Lys Ser Ser Asn
                85                  90                  95
aca gcc tac atg caa ctg agg agc ctg aca ttt gag gac tct tca gtc 336
Thr Ala Tyr Met Gln Leu Arg Ser Leu Thr Phe Glu Asp Ser Ser Val
            100                 105                 110
tat tac tgt gca aga gaa gag gat tac gac gga ggt cac tat gcc atg 384
Tyr Tyr Cys Ala Arg Glu Glu Asp Tyr Asp Gly Gly His Tyr Ala Met

115                 120                 125
gac tac tgg ggt caa ggc acc tca gtc acc gtc tcc tca                 423
Asp Tyr Trp Gly Gln Gly Thr Ser Val Thr Val Ser Ser
    130                 135                 140

<210>  30
<211>  141
<212>  PRT
<213>  Mus musculus
<400>  30
Met Glu Arg His Trp Ile Phe Leu Phe Leu Phe Ser Val Pro Ala Gly
1               5                   10                  15
Val Arg Ser Gln Val Gln Leu Gln Gln Ser Gly Ala Glu Leu Ala Lys
            20                  25                  30
Pro Gly Ala Ser Val Lys Met Ser Cys Lys Thr Ser Gly Tyr Thr Phe
        35                  40                  45
Thr Ala Tyr Trp Ile His Trp Val Lys Gln Arg Pro Gly Gln Gly Leu
    50                  55                  60
Glu Trp Ile Gly Tyr Ile Asn Pro Thr Thr Asp Tyr Thr Glu Tyr Asn
65                  70                  75                  80
Gln Arg Phe Lys Asp Lys Ala Thr Leu Thr Ala Asp Lys Ser Ser Asn
                85                  90                  95
Thr Ala Tyr Met Gln Leu Arg Ser Leu Thr Phe Glu Asp Ser Ser Val
            100                 105                 110
Tyr Tyr Cys Ala Arg Glu Glu Asp Tyr Asp Gly Gly His Tyr Ala Met
        115                 120                 125
Asp Tyr Trp Gly Gln Gly Thr Ser Val Thr Val Ser Ser
    130                 135                 140

<210>  31
<211>  642
<212>  DNA
<213>  Aspergillus fumigatus
<220>
<221>  CDS
<222>  (1)..(642)
<223>
<400>  31
atg cat cta ctg agc aaa gct gct gtc ctc ctc ctg gcg ctc aac gtc      48
Met His Leu Leu Ser Lys Ala Ala Val Leu Leu Leu Ala Leu Asn Val
1               5                   10                  15
agc gcc aga ccc atc gag gag cct cct gcg cag aca aca gcg ccc gcc      96
Ser Ala Arg Pro Ile Glu Glu Pro Pro Ala Gln Thr Thr Ala Pro Ala
            20                  25                  30
ggg tat gga gac tat aag gat tat ggc tcc tat ggc aag tac ggt gac     144
Gly Tyr Gly Asp Tyr Lys Asp Tyr Gly Ser Tyr Gly Lys Tyr Gly Asp
        35                  40                  45
tac ggt cac tac aag cgt gac gag gag ccc agc ccc acg acc aca tgc     192
Tyr Gly His Tyr Lys Arg Asp Glu Glu Pro Ser Pro Thr Thr Thr Cys
    50                  55                  60
acc gaa aca gaa act ccc act cca gca aac tac gga gac tac gga gac     240
Thr Glu Thr Glu Thr Pro Thr Pro Ala Asn Tyr Gly Asp Tyr Gly Asp
65                  70                  75                  80
tac ggc aac tat ggt caa tat ggg gac tat ggt cac tac aag cgt ggc     288
Tyr Gly Asn Tyr Gly Gln Tyr Gly Asp Tyr Gly His Tyr Lys Arg Gly
                85                  90                  95
gaa gag ccc act aca acc acc aca acc acc act gag acc cca agt ccc     336
Glu Glu Pro Thr Thr Thr Thr Thr Thr Thr Thr Glu Thr Pro Ser Pro
            100                 105                 110
tct cca gca aac tac gga aac tat ggt caa tat ggg gac tat ggt cac     384
Ser Pro Ala Asn Tyr Gly Asn Tyr Gly Gln Tyr Gly Asp Tyr Gly His
        115                 120                 125
tac aaa cgt gac gaa gag ccc acc gca acc acc act gag aca cca act     432

Tyr Lys Arg Asp Glu Glu Pro Thr Ala Thr Thr Thr Glu Thr Pro Thr
    130                 135                 140
ccc tct ccc act ccc gcc gac tac ggc aac tat gga gac tac ggc aac       480
Pro Ser Pro Thr Pro Ala Asp Tyr Gly Asn Tyr Gly Asp Tyr Gly Asn
145                 150                 155                 160
tac ggc aag tat ggg gac tac gga cag tac aaa gtg aag aga gac gac       528
Tyr Gly Lys Tyr Gly Asp Tyr Gly Gln Tyr Lys Val Lys Arg Asp Asp
                165                 170                 175
gag cct tgc acg acc acc acc gag acc ccg act ccc tct ccc act ccc       576
Glu Pro Cys Thr Thr Thr Thr Glu Thr Pro Thr Pro Ser Pro Thr Pro
            180                 185                 190
gcc gac tat ggt gac tat ggc aac tat ggc gac tat ggc aat tac ggc       624
Ala Asp Tyr Gly Asp Tyr Gly Asn Tyr Gly Asp Tyr Gly Asn Tyr Gly
        195                 200                 205
acc tac gag aac tac tag                                               642
Thr Tyr Glu Asn Tyr
    210

<210>  32
<211>  213
<212>  PRT
<213>  Aspergillus fumigatus
<400>  32
Met His Leu Leu Ser Lys Ala Ala Val Leu Leu Leu Ala Leu Asn Val
1               5                   10                  15
Ser Ala Arg Pro Ile Glu Glu Pro Pro Ala Gln Thr Thr Ala Pro Ala
            20                  25                  30
Gly Tyr Gly Asp Tyr Lys Asp Tyr Gly Ser Tyr Gly Lys Tyr Gly Asp
        35                  40                  45
Tyr Gly His Tyr Lys Arg Asp Glu Glu Pro Ser Pro Thr Thr Thr Cys
    50                  55                  60
Thr Glu Thr Glu Thr Pro Thr Pro Ala Asn Tyr Gly Asp Tyr Gly Asp
65                  70                  75                  80
Tyr Gly Asn Tyr Gly Gln Tyr Gly Asp Tyr Gly His Tyr Lys Arg Gly
                85                  90                  95
Glu Glu Pro Thr Thr Thr Thr Thr Thr Thr Thr Glu Thr Pro Ser Pro
            100                 105                 110
Ser Pro Ala Asn Tyr Gly Asn Tyr Gly Gln Tyr Gly Asp Tyr Gly His
        115                 120                 125
Tyr Lys Arg Asp Glu Glu Pro Thr Ala Thr Thr Thr Glu Thr Pro Thr
    130                 135                 140
Pro Ser Pro Thr Pro Ala Asp Tyr Gly Asn Tyr Gly Asp Tyr Gly Asn
145                 150                 155                 160
Tyr Gly Lys Tyr Gly Asp Tyr Gly Gln Tyr Lys Val Lys Arg Asp Asp
                165                 170                 175
Glu Pro Cys Thr Thr Thr Thr Glu Thr Pro Thr Pro Ser Pro Thr Pro
            180                 185                 190
Ala Asp Tyr Gly Asp Tyr Gly Asn Tyr Gly Asp Tyr Gly Asn Tyr Gly
        195                 200                 205
Thr Tyr Glu Asn Tyr
    210

<210>  33
<211>  33
<212>  PRT
<213>  Aspergillus fumigatus
<400>  33
Met His Leu Leu Ser Lys Ala Ala Val Leu Leu Leu Ala Leu Asn Val
1               5                   10                  15
Ser Ala Arg Pro Ile Glu Glu Pro Pro Ala Gln Thr Thr Ala Pro Ala
            20                  25                  30
Gly

<210> 34
<211> 40
<212> DNA
<213> Artificial
<220>
<223> Artificially synthesized primer sequence
<400> 34
taatacgact cactataggg cgcgcagctg taaacggtag 40

<210> 35
<211> 39
<212> DNA
<213> Artificial
<220>
<223> Artificially synthesized primer sequence
<400> 35
attaaccctc actaaaggga gggggtggac catcctcta 39

<210> 36
<211> 20
<212> DNA
<213> Artificial
<220>
<223> Artificially synthesized primer sequence
<400> 36
gataccctgg atgacttcag 20

<210> 37
<211> 20
<212> DNA
<213> Artificial
<220>
<223> Artificially synthesized primer sequence
<400> 37
ctctcagcat ggaaggacag 20

<210> 38
<211> 20
<212> DNA
<213> Artificial
<220>
<223> Artificially synthesized primer sequence
<400> 38
agggtacagt caccaagctg 20

<210> 39
<211> 20
<212> DNA
<213> Artificial
<220>
<223> Artificially synthesized primer sequence
<400> 39
tgcatgaggc tctccataac 20

<210> 40
<211> 20
<212> DNA
<213> Artificial
<220>
<223> Artificially synthesized primer sequence
<400> 40
tggacaggga tccagagttc 20

<210> 41
<211> 20
<212> DNA
<213> Artificial
<220>
<223> Artificially synthesized primer sequence
<400> 41
ctgctctgtg ttacatgagg 20

<210> 42
<211> 20
<212> DNA
<213> Artificial
<220>
<223> Artificially synthesized primer sequence
<400> 42
cactgccatc aatcttccac 20

<210> 43
<211> 20
<212> DNA
<213> Artificial
<220>
<223> Artificially synthesized primer sequence
<400> 43
tgtcaagagc ttcaacagga 20

<210> 44
<211> 20
<212> DNA
<213> Artificial
<220>
<223> Artificially synthesized primer sequence
<400> 44
gcactccgtt ctggataatg 20

<210> 45
<211> 48
<212> DNA
<213> Artificial
<220>
<223> Artificially synthesized primer sequence
<400> 45
ttttcctttt gcggccgccc cggcgggcgc tgttgtctgc gcaggagg 48

<210> 46
<211> 48
<212> DNA
<213> Artificial
<220>
<223> Artificially synthesized primer sequence
<400> 46
ttttcctttt gcggccgctg tggtcgtggg gctgggctcc tcgtcacg 48

<210> 47
<211> 48
<212> DNA
<213> Artificial
<220>
<223> Artificially synthesized primer sequence
<400> 47
ttttcctttt gcggccgcgt agtgaccata gtccccatat tgaccata 48

<210> 48
<211> 48
<212> DNA
<213> Artificial
<220>
<223> Artificially synthesized primer sequence
<400> 48
ttttcctttt gcggccgcat attgaccata gtttccgtag tttgctgg 48

<210> 49
<211> 48
<212> DNA
<213> Artificial
<220>
<223> Artificially synthesized primer sequence
<400> 49
ttttcctttt gcggccgcgc cgtagtcggc gggagtggga gagggagt 48

<210> 50
<211> 48
<212> DNA
<213> Artificial
<220>
<223> Artificially synthesized primer sequence
<400> 50
ttttcctttt gcggccgcgg tggtagtcgt gcgaggctcg tcgtctct 48

<210> 51
<211> 22
<212> DNA
<213> Artificial
<220>
<223> Artificially synthesized primer sequence
<400> 51
ggcgcgcagc tgtaaacggt ag 22

<210> 52
<211> 20
<212> DNA
<213> Artificial
<220>
<223> Artificially synthesized primer sequence
<400> 52
cgggggtgga ccatcctcta 20

<210> 53
<211> 1842
<212> DNA
<213> Aspergillus fumigatus
<220>
<221> CDS
<222> (601)..(1242)
<223>
<400> 53
cgagagtcaa cgagtgtgcg gagaccaacc aatctcgact ctgccgagaa cctcaacgct 60
gcaagtctga gacatgcaga acacaggcag ttgccgattc ccggttgttc gtatgttgag 120
tttctgatct cccgggtgac gaaaatgagt tccaatagga attggctctg gcggaacaga 180
gatggaaact ggtcagattg acattgcagt tccatggctg aatgtacagc ggacgaactg 240
ctcagatggc taatgtttgt gttacctgtg gcgacaaagg cttgggcagt gtaacaccgc 300
gtcgatagct tcctggcctg tattctgcag tatccctgat tggaacacca tggtgggttg 360
tattcgggcc atccagaccg ctgcagtcat cagagactta gccaataagc attgatccac 420
gacttggagg ttggggagtc atctttcggc tgtcgggctt aagtgcgaga tacagcaagg 480
aaatagagca gtgcactagc tataaagcct gctacccttg tccgtccaca aggtcttctt 540

catttcctgt cacagagcaa actcttctct tcatcctcaa caaccctatc atccagcacg 600
atg cat cta ctg agc aaa gct gct gtc ctc ctc ctg gcg ctc aac gtc 648
Met His Leu Leu Ser Lys Ala Ala Val Leu Leu Leu Ala Leu Asn Val
1               5                   10                  15
agc gcc aga ccc atc gag gag cct cct gcg cag aca aca gcg ccc gcc 696
Ser Ala Arg Pro Ile Glu Glu Pro Pro Ala Gln Thr Thr Ala Pro Ala
            20                  25                  30
ggg tat gga gac tat aag gat tat ggc tcc tat ggc aag tac ggt gac 744
Gly Tyr Gly Asp Tyr Lys Asp Tyr Gly Ser Tyr Gly Lys Tyr Gly Asp
        35                  40                  45
tac ggt cac tac aag cgt gac gag gag ccc agc ccc acg acc aca tgc 792
Tyr Gly His Tyr Lys Arg Asp Glu Glu Pro Ser Pro Thr Thr Thr Cys
    50                  55                  60
acc gaa aca gaa act ccc act cca gca aac tac gga gac tac gga gac 840
Thr Glu Thr Glu Thr Pro Thr Pro Ala Asn Tyr Gly Asp Tyr Gly Asp
65                  70                  75                  80
tac ggc aac tat ggt caa tat ggg gac tat ggt cac tac aag cgt ggc 888
Tyr Gly Asn Tyr Gly Gln Tyr Gly Asp Tyr Gly His Tyr Lys Arg Gly
                85                  90                  95
gaa gag ccc act aca acc acc aca acc acc act gag acc cca agt ccc 936
Glu Glu Pro Thr Thr Thr Thr Thr Thr Thr Thr Glu Thr Pro Ser Pro
            100                 105                 110
tct cca gca aac tac gga aac tat ggt caa tat ggg gac tat ggt cac 984
Ser Pro Ala Asn Tyr Gly Asn Tyr Gly Gln Tyr Gly Asp Tyr Gly His
        115                 120                 125
tac aaa cgt gac gaa gag ccc acc gca acc acc act gag aca cca act 1032
Tyr Lys Arg Asp Glu Glu Pro Thr Ala Thr Thr Thr Glu Thr Pro Thr
    130                 135                 140
ccc tct ccc act ccc gcc gac tac ggc aac tat gga gac tac ggc aac 1080
Pro Ser Pro Thr Pro Ala Asp Tyr Gly Asn Tyr Gly Asp Tyr Gly Asn
145                 150                 155                 160
tac ggc aag tat ggg gac tac gga cag tac aaa gtg aag aga gac gac 1128
Tyr Gly Lys Tyr Gly Asp Tyr Gly Gln Tyr Lys Val Lys Arg Asp Asp
                165                 170                 175
gag cct cgc acg act acc acc gag acc ccg act ccc tct ccc act ccc 1176
Glu Pro Arg Thr Thr Thr Thr Glu Thr Pro Thr Pro Ser Pro Thr Pro
            180                 185                 190
gcc gac tat ggt gac tat ggc aac tat ggc aac tat ggc aat tac ggc 1224
Ala Asp Tyr Gly Asp Tyr Gly Asn Tyr Gly Asn Tyr Gly Asn Tyr Gly
        195                 200                 205
acc tac gag aac tac tag cgggtcacag agggtcactg agcagaaaag 1272
Thr Tyr Glu Asn Tyr
    210
cgtaagttgt ctttaccttg attggaattg cccatcctga cgcattgcag ttcgagaaac 1332
gggccgacgg gggattaccg acatggaata tctgtggtat atcgagcaaa agctctgcgc 1392
agtttgcagt gatgttgatc atgattacac actccttaaa tcttaatgtc ttgtctagat 1452
aaaatgcatc ataacatacg cgagtcttag actctcatca gaaccagtac ttccactatc 1512
taccatgtaa ggagtaggaa aatccttgca taacaatata catcgacgac ggtagaaatg 1572
aaagcgaatg tgctgccctc ggtcaatgga gccaaaacgg attgaacatg atcgattcgc 1632
agagactgga tctcctgaaa aaaaaaaaaa aaagaaaaag agacaatatt attgtgtagg 1692
aatgcatgga ttgacatctg agcacctccg tgtggcggcg catggctcgt gatactatca 1752
cagcaatgct ccttgcatga atatgcaggg agccacactg atgtaacctc aaatgtacga 1812
ctgagccctg atcatttcgg atgcaccgac 1842

<210> 54
<211> 213
<212> PRT
<213> Aspergillus fumigatus
<400> 54
Met His Leu Leu Ser Lys Ala Ala Val Leu Leu Leu Ala Leu Asn Val
1               5                   10                  15
Ser Ala Arg Pro Ile Glu Glu Pro Pro Ala Gln Thr Thr Ala Pro Ala
            20                  25                  30

Gly Tyr Gly Asp Tyr Lys Asp Tyr Gly Ser Tyr Gly Lys Tyr Gly Asp
        35                  40                  45
Tyr Gly His Tyr Lys Arg Asp Glu Glu Pro Ser Pro Thr Thr Thr Cys
    50                  55                  60
Thr Glu Thr Glu Thr Pro Thr Pro Ala Asn Tyr Gly Asp Tyr Gly Asp
65                  70                  75                  80
Tyr Gly Asn Tyr Gly Gln Tyr Gly Asp Tyr Gly His Tyr Lys Arg Gly
                85                  90                  95
Glu Glu Pro Thr Thr Thr Thr Thr Thr Thr Thr Glu Thr Pro Ser Pro
            100                 105                 110
Ser Pro Ala Asn Tyr Gly Asn Tyr Gly Gln Tyr Gly Asp Tyr Gly His
        115                 120                 125
Tyr Lys Arg Asp Glu Glu Pro Thr Ala Thr Thr Thr Glu Thr Pro Thr
    130                 135                 140
Pro Ser Pro Thr Pro Ala Asp Tyr Gly Asn Tyr Gly Asp Tyr Gly Asn
145                 150                 155                 160
Tyr Gly Lys Tyr Gly Asp Tyr Gly Gln Tyr Lys Val Lys Arg Asp Asp
                165                 170                 175
Glu Pro Arg Thr Thr Thr Thr Glu Thr Pro Thr Pro Ser Pro Thr Pro
            180                 185                 190
Ala Asp Tyr Gly Asp Tyr Gly Asn Tyr Gly Asn Tyr Gly Asn Tyr Gly
        195                 200                 205
Thr Tyr Glu Asn Tyr
    210

<210>  55
<211>  1026
<212>  DNA
<213>  Escherichia coli
<220>
<221>  CDS
<222>  (1)..(1026)
<223>
<400>  55
atg aaa aag cct gaa ctc acc gcg acg tct gtc gag aag ttt ctg atc      48
Met Lys Lys Pro Glu Leu Thr Ala Thr Ser Val Glu Lys Phe Leu Ile
1               5                   10                  15
gaa aag ttc gac agc gtc tcc gac ctg atg cag ctc tcg gag ggc gaa      96
Glu Lys Phe Asp Ser Val Ser Asp Leu Met Gln Leu Ser Glu Gly Glu
            20                  25                  30
gaa tct cgt gct ttc agc ttc gat gta gga ggg cgt gga tat gtc ctg     144
Glu Ser Arg Ala Phe Ser Phe Asp Val Gly Gly Arg Gly Tyr Val Leu
        35                  40                  45
cgg gta aat agc tgc gcc gat ggt ttc tac aaa gat cgt tat gtt tat     192
Arg Val Asn Ser Cys Ala Asp Gly Phe Tyr Lys Asp Arg Tyr Val Tyr
    50                  55                  60
cgg cac ttt gca tcg gcc gcg ctc ccg att ccg gaa gtg ctt gac att     240
Arg His Phe Ala Ser Ala Ala Leu Pro Ile Pro Glu Val Leu Asp Ile
65                  70                  75                  80
ggg gaa ttc agc gag agc ctg acc tat tgc atc tcc cgc cgt gca cag     288
Gly Glu Phe Ser Glu Ser Leu Thr Tyr Cys Ile Ser Arg Arg Ala Gln
                85                  90                  95
ggt gtc acg ttg caa gac ctg cct gaa acc gaa ctg ccc gct gtt ctg     336
Gly Val Thr Leu Gln Asp Leu Pro Glu Thr Glu Leu Pro Ala Val Leu
            100                 105                 110
cag ccg gtc gcg gag gcc atg gat gcg atc gct gcg gcc gat ctt agc     384
Gln Pro Val Ala Glu Ala Met Asp Ala Ile Ala Ala Ala Asp Leu Ser
        115                 120                 125
cag acg agc ggg ttc ggc cca ttc gga ccg caa gga atc ggt caa tac     432
Gln Thr Ser Gly Phe Gly Pro Phe Gly Pro Gln Gly Ile Gly Gln Tyr
    130                 135                 140
act aca tgg cgt gat ttc ata tgc gcg att gct gat ccc cat gtg tat     480
Thr Thr Trp Arg Asp Phe Ile Cys Ala Ile Ala Asp Pro His Val Tyr

145                 150                 155                 160
cac tgg caa act gtg atg gac gac acc gtc agt gcg tcc gtc gcg cag      528
His Trp Gln Thr Val Met Asp Asp Thr Val Ser Ala Ser Val Ala Gln
                165                 170                 175
gct ctc gat gag ctg atg ctt tgg gcc gag gac tgc ccc gaa gtc cgg      576
Ala Leu Asp Glu Leu Met Leu Trp Ala Glu Asp Cys Pro Glu Val Arg
            180                 185                 190
cac ctc gtg cac gcg gat ttc ggc tcc aac aat gtc ctg acg gac aat      624
His Leu Val His Ala Asp Phe Gly Ser Asn Asn Val Leu Thr Asp Asn
        195                 200                 205
ggc cgc ata aca gcg gtc att gac tgg agc gag gcg atg ttc ggg gat      672
Gly Arg Ile Thr Ala Val Ile Asp Trp Ser Glu Ala Met Phe Gly Asp
    210                 215                 220
tcc caa tac gag gtc gcc aac atc ttc ttc tgg agg ccg tgg ttg gct      720
Ser Gln Tyr Glu Val Ala Asn Ile Phe Phe Trp Arg Pro Trp Leu Ala
225                 230                 235                 240
tgt atg gag cag cag acg cgc tac ttc gag cgg agg cat ccg gag ctt      768
Cys Met Glu Gln Gln Thr Arg Tyr Phe Glu Arg Arg His Pro Glu Leu
                245                 250                 255
gca gga tcg ccg cgg ctc cgg gcg tat atg ctc cgc att ggt ctt gac      816
Ala Gly Ser Pro Arg Leu Arg Ala Tyr Met Leu Arg Ile Gly Leu Asp
            260                 265                 270
caa ctc tat cag agc ttg gtt gac ggc aat ttc gat gat gca gct tgg      864
Gln Leu Tyr Gln Ser Leu Val Asp Gly Asn Phe Asp Asp Ala Ala Trp
        275                 280                 285
gcg cag ggt cga tgc gac gca atc gtc cga tcc gga gcc ggg act gtc      912
Ala Gln Gly Arg Cys Asp Ala Ile Val Arg Ser Gly Ala Gly Thr Val
    290                 295                 300
ggg cgt aca caa atc gcc cgc aga agc gcg gcc gtc tgg acc gat ggc      960
Gly Arg Thr Gln Ile Ala Arg Arg Ser Ala Ala Val Trp Thr Asp Gly
305                 310                 315                 320
tgt gta gaa gta ctc gcc gat agt gga aac cga cgc ccc agc act cgt     1008
Cys Val Glu Val Leu Ala Asp Ser Gly Asn Arg Arg Pro Ser Thr Arg
                325                 330                 335
ccg agg gca aag gaa tag                                             1026
Pro Arg Ala Lys Glu
            340

<210>  56
<211>  341
<212>  PRT
<213>  Escherichia coli
<400>  56
Met Lys Lys Pro Glu Leu Thr Ala Thr Ser Val Glu Lys Phe Leu Ile
1               5                   10                  15
Glu Lys Phe Asp Ser Val Ser Asp Leu Met Gln Leu Ser Glu Gly Glu
            20                  25                  30
Glu Ser Arg Ala Phe Ser Phe Asp Val Gly Gly Arg Gly Tyr Val Leu
        35                  40                  45
Arg Val Asn Ser Cys Ala Asp Gly Phe Tyr Lys Asp Arg Tyr Val Tyr
    50                  55                  60
Arg His Phe Ala Ser Ala Ala Leu Pro Ile Pro Glu Val Leu Asp Ile
65                  70                  75                  80
Gly Glu Phe Ser Glu Ser Leu Thr Tyr Cys Ile Ser Arg Arg Ala Gln
                85                  90                  95
Gly Val Thr Leu Gln Asp Leu Pro Glu Thr Glu Leu Pro Ala Val Leu
            100                 105                 110
Gln Pro Val Ala Glu Ala Met Asp Ala Ile Ala Ala Ala Asp Leu Ser
        115                 120                 125
Gln Thr Ser Gly Phe Gly Pro Phe Gly Pro Gln Gly Ile Gly Gln Tyr
    130                 135                 140
Thr Thr Trp Arg Asp Phe Ile Cys Ala Ile Ala Asp Pro His Val Tyr
145                 150                 155                 160

His Trp Gln Thr Val Met Asp Asp Thr Val Ser Ala Ser Val Ala Gln
                165                 170                 175
Ala Leu Asp Glu Leu Met Leu Trp Ala Glu Asp Cys Pro Glu Val Arg
            180                 185                 190
His Leu Val His Ala Asp Phe Gly Ser Asn Asn Val Leu Thr Asp Asn
        195                 200                 205
Gly Arg Ile Thr Ala Val Ile Asp Trp Ser Glu Ala Met Phe Gly Asp
    210                 215                 220
Ser Gln Tyr Glu Val Ala Asn Ile Phe Phe Trp Arg Pro Trp Leu Ala
225                 230                 235                 240
Cys Met Glu Gln Gln Thr Arg Tyr Phe Glu Arg Arg His Pro Glu Leu
                245                 250                 255
Ala Gly Ser Pro Arg Leu Arg Ala Tyr Met Leu Arg Ile Gly Leu Asp
            260                 265                 270
Gln Leu Tyr Gln Ser Leu Val Asp Gly Asn Phe Asp Asp Ala Ala Trp
        275                 280                 285
Ala Gln Gly Arg Cys Asp Ala Ile Val Arg Ser Gly Ala Gly Thr Val
    290                 295                 300
Gly Arg Thr Gln Ile Ala Arg Arg Ser Ala Ala Val Trp Thr Asp Gly
305                 310                 315                 320
Cys Val Glu Val Leu Ala Asp Ser Gly Asn Arg Arg Pro Ser Thr Arg
                325                 330                 335
Pro Arg Ala Lys Glu
            340